# EUROPEAN PATENT SPECIFICATION

(11) **EP 2 785 861 B1**
(45) Date of publication and mention of the grant of the patent: **15.01.2020**
(21) Application number: 12787697.7
(22) Date of filing: 12.11.2012
(51) Int. Cl.: C12Q 1/68, C12Q 1/6886

(54) **METHODS AND KITS FOR THE PROGNOSIS OF COLORECTAL CANCER**
VERFAHREN UND KITS FÜR DIE PROGNOSE VON KOLOREKTALEM KREBS
PROCÉDÉS ET KITS POUR LE PRONOSTIC DU CANCER COLORECTAL

(30) Priority: 28.11.2011 EP 11382368
(43) Date of publication of application: 08.10.2014
(73) Proprietor: Institució Catalana de Recerca i Estudis Avançats, 08010 Barcelona (ES); Fundació Institut de Recerca Biomèdica (IRB Barcelona), 08028 Barcelona (ES)
(72) Inventor: BATLLE GÓMEZ, Eduard, E-08028 Barcelona (ES); SANCHO SUILS, Elena, E-08028 Barcelona (ES); ROSSELL RIBERA, David, E-08360 Canet de Mar (ES); CALON, Alexandre, F-67205 Oberhausbergen (FR); ESPINET HERNÁNDEZ, Elisa, E-47014 Valladolid (ES); PALOMO PONCE, Sergio, E-08950 Esplugues de Llobregat (ES)
(74) Representative: ABG Intellectual Property Law, S.L.
(86) International application number: PCT/EP2012/072425
(87) International publication number: WO 2013/079309

(56) References cited:
- WO-A2-2006/135886
- US-A1- 2007 238 115
- R. N. JORISSEN ET AL: "Metastasis-Associated Gene Expression Changes Predict Poor Outcomes in Patients with Dukes Stage B and C Colorectal Cancer", CLINICAL CANCER RESEARCH, vol. 15, no. 24, 8 December 2009 (2009-12-08), pages 7642-7651, XP055039833, ISSN: 1078-0432, DOI: 10.1158/1078-0432.CCR-09-1431
- "Affymetrix Human Genome U133 Plus 2.0 Array [CDF: HGU133Plus2_Hs_ENSG v12.1.0]", GEO , 19 April 2010 (2010-04-19), XP002668741, Retrieved from the Internet: URL:http://www.ncbi.nlm.gov/geo/quera/acc. cgi?acc=GPL9987 [retrieved on 2010-04-19]
- R. SALAZAR ET AL: "Gene Expression Signature to Improve Prognosis Prediction of Stage II and III Colorectal Cancer", JOURNAL OF CLINICAL ONCOLOGY, vol. 29, no. 1, 22 November 2010 (2010-11-22), pages 17-24, XP055032270, ISSN: 0732-183X, DOI: 10.1200/JCO.2010.30.1077
- DATABASE MEDLINE [Online] US NATIONAL LIBRARY OF MEDICINE (NLM), BETHESDA, MD, US; 10 December 2011 (2011-12-10), GRAY RICHARD G ET AL: "Validation study of a quantitative multigene reverse transcriptase-polymerase chain reaction assay for assessment of recurrence risk in patients with stage II colon cancer.", XP002679585, Database accession no. NLM22067390 & JOURNAL OF CLINICAL ONCOLOGY : OFFICIAL JOURNAL OF THE AMERICAN SOCIETY OF CLINICAL ONCOLOGY 10 DEC 2011 LNKD- PUBMED:22067390, vol. 29, no. 35, 10 December 2011 (2011-12-10), pages 4611-4619, ISSN: 1527-7755

### SEQUENCE LISTING

<110> FUNDACIÓ PRIVADA INSTITUT DE RECERCA BIOMÈDICA FUNDACIÓ PRIVADA INSTITUCIÓ CATALANA DE RECERCA I ESTUDIS AVANÇATS
<120> METHODS AND KITS FOR THE PROGNOSIS OF COLORECTAL CANCER
<130> P5057PC00
<150> EP11382368
   <151> 2011-11-28
<160> 13
<170> PatentIn version 3.5
<210> 1
   <211> 271
   <212> DNA
   <213> Artificial
<220>
   <223> Affymetrix 1553275_s_at probe
<220>
   <221> misc_feature
   <222> (229)..(229)
   <223> n is a, c, g, or t
<400> 1
   aaagcagctt cctacacaat gcagccccat acccataccc aacccattga cccatcaagc      60
   cagcacccat cctttcttgg cactctgtat ctcagtacac attctacctt caaaaacagc     120
   taagtcaaac gtgtcaaaat agctatttcc tcttgagttt gagcatctgc agttgcccag     180
   ccccaattct ttatttggaa ataagtatta ggagaaacat atctagcana gtgatattga     240
   tttgtaattt cctcaaatag acattcactt t                                    271
<210> 2
   <211> 468
   <212> DNA
   <213> Artificial
<220>
   <223> Affymtrix "1556773_at probe
<220>
   <221> misc_feature
   <222> (38)..(39)
   <223> n is a, c, g, or t
<220>
   <221> misc_feature
   <222> (168)..(168)
   <223> n is a, c, g, or t
<400> 2
   ggagctcgat tcacgtacag cacttctgtg gggtttgnna aaaaaaaaag gaaaacaaca       60
   gaagaacaca tcatatgcaa ctaatgatct cattatttaa gagtcccctg ttacttcttt      120
   agtcatttcc tttgactctg ctacagatag gattatagga tgatgctnca aaggggacct      180
   tgaacctatt caccattatt tgtctcttta agctggcaaa cccatcatta aatagcacat      240
   aaaatagcaa tcatatggga taagtagtac agcttcagta atcaatgggc agtggcacta      300
   gaaaaatctt gagcacagtg aatgacctat cctgcaaaca tctaatggat ctctaaaggg      360
   taacaaaccc tataaattct ggcttactgc acatatttag tgtgttttaa gataggatcc      420
   taactgtata tatttataac taatgtaagg accctacttt attgccaa                   468
<210> 3
   <211> 471
   <212> DNA
   <213> Affymtrix 225227_at probe
<400> 3
   gtaggacatc ttttgaggaa ttagtttatg agaaataaaa ttttacttgt ttttactatc       60
   ctgttagaag tatttgttta tcctgataat tttaagccaa catagtagtc ttaaattact      120
   tttgaatttc taatctgtga aggcagtaaa tgaaatatct gttctgcaac tgttgaaaca      180
   aataattggc tacattgacc ataattaaag ttaaaatttt gccaatgatg tacagtttta      240
   tggttaaagt tgctgtggtt ggttgcatta catgacacag aaaactgtcc tctacctcac      300
   gtgaaataaa tattttatat ggttttacta aaaataagac tcatgtatct ggtcacctag      360
   tttacaaatt ttgaattata tttattgaaa catgacatac tgtgctctga gcttatacct      420
   caattgtatt ttgtgctgtt ttccattttc atgccttgta aataacttgt a               471
<210> 4
   <211> 417
   <212> DNA
   <213> Artificial
<220>
   <223> Affymetrix 225328_at probe
<400> 4
   gaatctctca aaacttaggc cctgctccat ttggccaaaa atgatggctg ctccaaaact        60
   ctgaacttct taaaactcca tctgctacat tatttctgga gtttaaacat gactttttct       120
   gtctttgagt atagatgtgt ttgtttaatt aacgaagcac aagtctgtta agcagaaggc       180
   tccaagctgt attctatact tgggaatcct tggtgccatc tttattctac caagtgccaa       240
   tcaccatggc taaagtgggc gtatattaca gcctgtgtcc taagcttaga agctttaatg       300
   tactttttta aatgaaaagt attagagggg gttgaacatt gtaactaaag cataaagtta       360
   gaccaattac atgcagagat gtttatttaa tattgtgtga gctgagtcct tctgtat          417
<210> 5
   <211> 414
   <212> DNA
   <213> Artificial
<220>
   <223> Affymetrix 232453_at probe
<220>
   <221> misc_feature
   <222> (236)..(236)
   <223> n is a, c, g, or t
<400> 5
   gttaccatac cgtatgcttg gcactcagta caccaagaaa acaaagaggc attgcctgtc        60
   tcgaagttgc ttagagtcta gtgggggaga taatgcacac ctgcatccat agcacagcaa       120
   tgtgtacata ataacaaaga tctgaaaacc ccagtcagtt ttctatgttt tgtcctcttt       180
   cacacccttc cgagagttct atctaatgta actatgaatg gtcactgtgt ctttantttt       240
   ggcccagact cttctcaatg cttcagcctc acgtatccaa ctacctactg attattctgc       300
   ttggacatac atcagccatt ttaaatttgg cctgagttga actcattctc tttctctccc       360
   acattcattg tgttttcatt tgttttcttg aaatctcctg gttaagtcag ttca             414
<210> 6
   <211> 534
   <212> DNA
   <213> Artificial
<220>
   <223> Affymetrix 232544_at
<400> 6
   gaagactttc ctgaatttat tttgcctata ttttgtttct gatgaatgag caagggggtt      60
   atatgatata tttgtgtcta aatacatcta aaagagctct ttcattcgca taaaataaac    120
   atgctgtatg ataacaaagt ttatgtcaga gttgagatgg ctctttatgg tacatatatg    180
   ctgtgccttc taaaaaatgt catattatat tcagtgaaag atgatgtttc ttaaaatgaa    240
   tgaatgtctc tgaggtggcc aacattagtc cttctatagc cagattagca atcttagagc    300
   ctggtcaggc acagtggctc ttgtttgtaa tcccagtact ttgggaggcc aaggtgggag    360
   gatctcttga gctcaggagt tcaggaccag cctgggcaac atagtgagat ctcatctcta    420
   ctaaaaataa aaatgaaatt gttaattggc caggcacagt gggaagcacc tgtagtccca    480
   gctactcagg aggctaaagt gagagggtgg ctctagcaca gtcatcaagg ctgc          534
<210> 7
   <211> 442
   <212> DNA
   <213> Artificial
<220>
   <223> Affymetrix 235629_at probe
<400> 7
   tgacttttag atattccaca gaaccagact tatttgatgt ggataataac caatgattta     60
   gcattttgtt tgcttttgtt ttattttatc cgggttcatt ttttactctt cccatgtaca    120
   tgaaacaggt ggtggcgtgt agagatcagc tgatccttgt tttatggtta attgaactac    180
   tttgtatcca gggtttctgc aaatccaaaa gtgatttttc atctaggatc tattcctaac    240
   agtctactcc aatcccactt tagttttcca caattttaaa tcttaatagt gagaattcaa    300
   atgaaagtca tttcatttga ctattctgat gacatgattg tggcagaata aattgggtct    360
   taaaatgccc tagaaaatgg taaatgataa aaaataatat tttaaaattc aaccaaagaa    420
   atggcccatt ggccaggtgt gg                                             442
<210> 8
   <211> 407
   <212> DNA
   <213> Artificial
<220>
   <223> Affymetrix 236764_at probe
<220>
   <221> misc_feature
   <222> (381)..(381)
   <223> n is a, c, g, or t
<400> 8
   gtttgtgatg gcatcagtct cctcatttaa gactgacatt taagctgata atttagatcc       60
   tgtctgcttg gaggcattat tattatttta ctgggaggct cggctaagaa ggtaaaagga      120
   cacaaagccc tgtagccttc tcctgggagc atggattatt ttttcagatt ccctaagtgc      180
   tgggagatgg atgagtacat tttaagggca cttggaagta ggatagaagg agtgaggtgg      240
   gatctattct cctaagtacc aaggctgtag aaccataagt tagggacaga ggagaccact      300
   ccagaacaga gcattaaaaa ggaacaagag gaggaatagc aaagaaaaaa atgtctcctt      360
   tctgaatttt gctaaatgct ngccccagcc ccaagtggaa aaagcat                    407
<210> 9
   <211> 404
   <212> DNA
   <213> Artificial
<220>
   <223> Affymterix 237452_at probe
<220>
   <221> misc_feature
   <222> (196)..(196)
   <223> n is a, c, g, or t
<400> 9
   ggtttgagga tcacgacctt caattaaagt ccagagaaac agctctgagg gatgggcaga       60
   gggacagctt caatccttcc tgcccagccc tggggcctcc tgaaagctta acctcttggc      120
   tccaaggtgg agaggtgttc atctcctcca ctcctttaaa tatggagctc attcctggaa      180
   ggggcttcct caatcnggaa ggactgatgc tggggatggc catagaggaa tttggccgca      240
   ccacgaaacc ccgtcagtgg cgtgagagtc acacacgatg ggcttcctca ccaggcctgc      300
   aacatctgtt gggggaagaa aggaattaaa acagggggag gcctggattt ttgaaatctt      360
   tccagtgtca ttcgagcgca ttctgtggga agcccgggag tttg                       404
<210> 10
   <211> 443
   <212> DNA
   <213> Artificial
<220>
   <223> Affymetrix 238617_at probe
<220>
   <221> misc_feature
   <222> (30)..(30)
   <223> n is a, c, g, or t
<220>
   <221> misc_feature
   <222> (182)..(182)
   <223> n is a, c, g, or t
<220>
   <221> misc_feature
   <222> (203)..(203)
   <223> n is a, c, g, or t
<400> 10
   cccaggtgca tttattgtgc cagaaacagn gaattcctaa ataagttcat aaaatcttgt      60
   tttacaattc aatggtagtg cttaatgtta ctgtctgaaa tccactgttt aaccagatgg     120
   aagactgggg ccattcaaaa cattattagt tcacaagtgt ttgctgttaa aatgcttcaa     180
   tnaaactcat ttgttaaagt canaaattca actcttctgt acttgggcag gtcccagcag     240
   ccccttggca ggctagtttt gtgcatgggc ctttgcaagc tatataaacg ttaggtgttg     300
   gcttgattat gataaatatg agcaggctcc ctggagaaca gtccagaaaa aaagagttat     360
   agttctcaca aactggtgac atgaatccat taagctgtgt ggcattttcc agtgtaaaga     420
   cattgtttgc tggctacgat ctc                                             443
<210> 11
   <211> 528
   <212> DNA
   <213> Artificial
<220>
   <223> Affymetrix 240135_x_at probe
<220>
   <221> misc_feature
   <222> (116)..(116)
   <223> n is a, c, g, or t
<220>
   <221> misc_feature
   <222> (159)..(159)
   <223> n is a, c, g, or t
<220>
   <221> misc_feature
   <222> (163)..(163)
   <223> n is a, c, g, or t
<220>
   <221> misc_feature
   <222> (168)..(168)
   <223> n is a, c, g, or t
<220>
   <221> misc_feature
   <222> (205)..(205)
   <223> n is a, c, g, or t
<220>
   <221> misc_feature
   <222> (226)..(226)
   <223> n is a, c, g, or t
<220>
   <221> misc_feature
   <222> (264)..(264)
   <223> n is a, c, g, or t
<220>
   <221> misc_feature
   <222> (341)..(341)
   <223> n is a, c, g, or t
<220>
   <221> misc_feature
   <222> (344)..(344)
   <223> n is a, c, g, or t
<220>
   <221> misc_feature
   <222> (355)..(355)
   <223> n is a, c, g, or t
<220>
   <221> misc_feature
   <222> (395)..(395)
   <223> n is a, c, g, or t
<220>
   <221> misc_feature
   <222> (434)..(434)
   <223> n is a, c, g, or t
<400> 11
   agatcctttt gggcagcctg aagactgggg agggttttcc tccctcctcc acctccaccc      60
   acattccact taccctttcc ctcctcatcc cagttggtaa cattcccacc tgattnagtg     120
   tggggagttg ctgctgcagc tgagttctgg ttttgcccna gcnaggantc acacacgtgc     180
   ggccaggagg ctggcaggtg gaatncaggg tgtgcagggg tgcagnccca agtccttttc     240
   tgctggaaaa tgggctgggt gacnttcact tgcgtttttt tggtatgtag tgggagtagg     300
   tccccattct tcccccttgc cctttgcttt ggggtttcta nccntaaaaa ttganccacg     360
   gaaaagtgaa aacaagcatg agtagatcct gcttnccaat gggggccttc ccgtctgctt     420
   actgcttagg tttnctgcgg tgggtctgag caatgcagaa agtttcttac aatttctctg     480
   gattgaaaca agaaatagct ccctcgctcc tcttcataaa tttctcag                 528
<210> 12
   <211> 427
   <212> DNA
   <213> Artificial
<220>
   <223> Affymetrix 241272_at probe
<220>
   <221> misc_feature
   <222> (204)..(205)
   <223> n is a, c, g, or t
<220>
   <221> misc_feature
   <222> (216)..(216)
   <223> n is a, c, g, or t
<220>
   <221> misc_feature
   <222> (224)..(224)
   <223> n is a, c, g, or t
<220>
   <221> misc_feature
   <222> (227)..(228)
   <223> n is a, c, g, or t
<220>
   <221> misc_feature
   <222> (260)..(260)
   <223> n is a, c, g, or t
<220>
   <221> misc_feature
   <222> (264)..(264)
   <223> n is a, c, g, or t
<220>
   <221> misc_feature
   <222> (272)..(272)
   <223> n is a, c, g, or t
<220>
   <221> misc_feature
   <222> (275)..(276)
   <223> n is a, c, g, or t
<220>
   <221> misc_feature
   <222> (281)..(281)
   <223> n is a, c, g, or t
<220>
   <221> misc_feature
   <222> (288)..(288)
   <223> n is a, c, g, or t
<220>
   <221> misc_feature
   <222> (298)..(298)
   <223> n is a, c, g, or t
<220>
   <221> misc_feature
   <222> (327)..(327)
   <223> n is a, c, g, or t
<220>
   <221> misc_feature
   <222> (401)..(401)
   <223> n is a, c, g, or t
<400> 12
   taactgggca ccagctaagc cactgtgctg cctaaatgga caaccacttc tcagttctgg     60
   aattcttcaa acacctaggg ttaggagggt cttcgctttg gcagttagag ttttctcaat    120
   tcttttatga cacaggttgc accggactca ctcaactatg gagatcagat gtcctcagga    180
   aaaaaaaaaa aaaaaaaaaa aaanntacac acttanggct gttnccnngg ctcctctctc    240
   ttcctgacac attccaaaan ggantttatg gntgnnggga nggacaangg gtgaggantt    300
   tctaggacat ttaagaacgc ccatgtnagc aactctatgc ttgacaggcc atgcacaata    360
   aaactctttg gcctgtaagc gagaagaaaa atttggtgta ngctgcctca tgggttgcca    420
   aaatgat                                                              427
<210> 13
   <211> 321
   <212> DNA
   <213> Artificial
<220>
   <223> Affymetrix 243416_at probe
<220>
   <221> misc_feature
   <222> (142)..(142)
   <223> n is a, c, g, or t
<220>
   <221> misc_feature
   <222> (146)..(146)
   <223> n is a, c, g, or t
<220>
   <221> misc_feature
   <222> (201)..(201)
   <223> n is a, c, g, or t
<400> 13
   gagctctgtc ctatccagag aggctaaacc aacttggatg gctcaagaca atatagaaat        60
   aaacgctaaa atatgggatc tagagtacac ccagggttgc tatatcgaat tcatgtttct       120
   agaggtcagt tcaacacaat gnatgnaagt ggaccaggtg taggtaagca gggagtggtg       180
   ctggatgttg caagctggag nacatgtgcc aagggtgaag gggggcagga ccactcagtt       240
   actctgttgc ccagtagcca tgtgggtcca atgttgtcac atgactttgt tttttatgag       300
   aagctgggtg tctgtttttt c                                                 321

## Description

### FIELD OF THE INVENTION

The invention relates to the field of diagnosis and, more in particular, to methods for predicting the risk of relapse of cancer patients as well as methods for providing personalized medicine to said patients. The invention relates as well to kits for carrying out the diagnostic and predictive medicine methods.

### BACKGROUND OF THE INVENTION

Colorectal cancer (CRC) is one of the most frequent neoplasias in the western world, it is the third cause of death in men, after lung cancer and prostate cancer and it is the second in frequency among women, after breast cancer. Colorectal cancer is the third most common cancer in men (663 000 cases, 10.0% of the total) and the second in women (571 000 cases, 9.4% of the total) worldwide. About 608 000 deaths from colorectal cancer are estimated worldwide, accounting for 8% of all cancer deaths, making it the fourth most common cause of death from cancer. (GLOBOCAN.iarc.fr)

The main treatment option for colorectal cancer is surgery, with or without adjuvant chemotherapy and/or radiotherapy, depending on the individual patient's staging and other medical factors.

The selection of an appropriate treatment is crucial both for the patient and for economical reasons. For patient survival, it is essential to know when to use immediately a heavy and aggressive treatment protocol in order to prevent extension of a malignant colorectal cancer. Otherwise, survival of the patient may be compromised. In contrast, performing a heavy and aggressive treatment when it is not necessitated is highly disadvantageous for the patient. Such treatments subject patients to a degree of discomfort and inconvenience derived from adverse toxicities that may significantly affect the patient's quality of life. Of note, each patient incurs a one in 400 chance that the therapy will result in fatal toxicity. In addition, heavy and aggressive treatments are usually very costly, and thus they should be performed only when necessary.

Currently, treatment selection is based on tumor staging, which is usually performed using the Tumor/Node/Metastasis (TNM) test from the American Joint Committee on Cancer (AJCC). The TNM system assigns a number based on three categories. "T" denotes the degree of invasion of the intestinal wall, "N" the degree of lymphatic node involvement, and "M" the degree of metastasis. The broader stage of a cancer is usually quoted as a number I, II, III, IV derived from the TNM value grouped by prognosis; a higher number indicates a more advanced cancer and likely a worse outcome. Details of this system are in Table 1 below:

| **AJCC stage** | **TNM stage** | **TNM stage criteria for colorectal cancer** |
|---|---|---|
| Stage 0 | Tis N0 M0 | Tis: Tumor confined to mucosa; cancer-*in-situ* |
| Stage I | T1 N0 M0 | T1: Tumor invades submucosa |
| Stage I | T2 N0 M0 | T2: Tumor invades muscularis propria |
| Stage II-A | T3 N0 M0 | T3: Tumor invades subserosa or beyond (without other organs involved) |
| Stage II-B | T4 N0 M0 | T4: Tumor invades adjacent organs or perforates the visceral peritoneum |
| Stage III-A | T1-2 N1 M0 | N1: Metastasis to 1 to 3 regional lymph nodes. T1 or T2. |
| Stage III-B | T3-4 N1 M0 | N1: Metastasis to 1 to 3 regional lymph nodes. T3 or T4. |
| Stage III-C | any T, N2 M0 | N2: Metastasis to 4 or more regional lymph nodes. Any T. |
| Stage IV | any T, any N, M1 | M1: Distant metastases present. Any T, any N. |

Although the AJCC classification provides some valuable information concerning the stage at which colorectal cancer has been diagnosed, it does not give information on the tumor aggressiveness and its usefulness for prognosis is limited. Whereas it is clear that patients at stage IV have bad prognosis, diagnosis of colorectal cancer at an early stage does not preclude the possibility that the tumor may further develop very rapidly. In particular, it is totally unknown why 20 to 40 percent of patients with stage II colorectal cancer (i.e., early cancer with neither metastasis nor lymph node invasion at diagnosis) will rapidly worsen and die. Some studies suggest that a subset of patients with high-risk stage II colon cancer may benefit from adjuvant therapy (Quasar collaborative group et al., Lancet 2007; 370:2020-2029). Yet, histopathological variables, such as high-risk features in stage II disease, are only directive when stratifying therapy. When lymph nodes are invaded by tumor cells, the TNM test scores as bad prognosis and the patient is usually subjected to surgery followed by heavy chemotherapy. Clinical studies show that for every 25 patients identified as high-risk stage II CRC, 20 will cure regardless of whether they receive treatment or not (Quasar collaborative group et al., Lancet 2007; 370:2020-2029). Likewise, a subset of patients with stage III colon cancer treated only by surgery did not recur in 5 years even without adjuvant treatment (Ranghammar et al., Acta Oncologica 2001; 40: 282-308). Adjuvant chemotherapy is standard recommendation for stage III CRC, yet prospective identification of this subgroup of patients with stage III colon cancer could spare therapy. Thus, an accurate and reliable method that identifies patients at greatest and least risk (eg, "high-risk" stage II and "low-risk" stage III colon cancer) could improve the selection of individualized therapy within these groups.

For this reason, several methods for predicting the outcome of patients suffering colorectal cancer based on the expression levels of molecular markers have been described.

Jorissen et al. (Clin. Cancer Res., 2009, 15 :7642-7651) have described a classifier formed by 128 genes which show reproducible variations between patients suffering stage A CRC (corresponding to stage I) and stage D (corresponding to stage IV). Moreover, at least two genes of the classifier (NPR3/C5orf23 y FLT1) are up-regulated in patients which suffered recurrence of the disease.

WO2010042228 describe the identification of a signature formed by 176 genes, the expression levels of which correlate with the prognosis of CRC.

WO2010124222 describes that colon cancer patients wherein the expression levels of FLT-1 (also known as VEGFR-1) are higher than a reference value show a higher probability of showing recurrence of the tumor after surgical resection.

US7695913 describes a method for predicting the prognosis of a patient suffering CRC which comprises the determination of the normalized expression levels of the INHBA, MYBL2, FAP and Ki67 genes wherein an increased expression of INHBA and FAP negatively correlates with an increased probability of a positive prognosis and wherein the expression of the MYBL2 and Ki67 genes positively correlates with an increased possibility of positive prognosis. The method described in this document forms the basis of the Oncotype DX kit although the kit includes the determination of 12 genes, including the INHBA, MYBL2, FAP and Ki67 genes.

WO02057787 reports the results of a study designed to determine whether Survivin mRNA can be used to predict death from recurrent colorectal carcinoma. The study was based on data obtained from frozen tumour biopsies from 144 patients. The study reportedly shows that Survivin expression is associated with a significantly greater risk of death due to recurrent cancer in patients with stage II colorectal cancer.

Rosati et al. (Tumour Biol, 2004, 25:258-63) reports the results of a study designed to determine whether expression of thymidylate synthase (TS), p53, bcl-2, Ki-67 and p27 protein in colorectal adenocarcinoma is predictive of disease free survival or overall survival. Specimens from 103 patients were examined by immunohistochemistry. According to this reference, there is no statistically significant association between the expression of any of TS, p53, bcl-2, Ki-67 and p27 and a clinical outcome although a statistically significant association between an unfavourable outcome and a combination of p53-negative expression, Ki-67 positive expression and stage C cancers was observed.

Nevertheless, despite the research carried out on this topic, today there are very few tumor markers which are useful from the clinical point of view both for the diagnosis of CRC and for determining the stage of a CRC carcinoma. A test capable of quantifying likelihood of patient benefit from chemotherapy to identify more accurately Stage III patients for treatment would be extremely useful. A patient having a low recurrence risk resembling that of a Stage II patient and a low likelihood of benefit from chemotherapy might elect to forego chemotherapy. A patient with a high recurrence risk and a low likelihood of benefit from 5-FU based chemotherapy might elect an alternative treatment.

Therefore, there is a need in the art for markers or panels of markers which allow the diagnosis of CRC and the classification of the stage of colorectal carcinomas with a high reliability.

Thus, an accurate and reliable method that identifies patients at greatest and least risk (e.g., "high-risk" stage II and "low-risk" stage III colon cancer) could improve the selection of individualized therapy within these groups.

### SUMMARY OF THE INVENTION

In a first aspect, the invention relates to a method for predicting the outcome of a patient suffering colorectal cancer, or for selecting a patient which is likely to benefit from adjuvant therapy after surgical resection of colorectal cancer comprising the determination of the expression levels of the NPR3/C5orf23, CDKN2B and FLT1 genes in a sample from said patient,
wherein an increased expression level of said genes with respect to a reference value for said genes is indicative of an increased likelihood of a negative outcome of the patient, or that the patient is likely to benefit from therapy after surgical treatment or
wherein a decreased expression level of said genes with respect to reference values for said genes is indicative of an increased likelihood of a positive outcome of the patient, or that the patient is unlikely to benefit from therapy after surgical treatment.

In another aspect, the invention relates to a chemotherapeutic agent for use in the treatment of colorectal cancer in a patient after surgical treatment of the cancer, wherein the patient has been selected by a method according to the first aspect of the invention and wherein the chemotherapeutic agent comprises a TGF-beta inhibitor.

In another aspect, the invention relates to a kit comprising reagents adequate for determining the expression levels of the NPR3/C5orf23, CDKN2B and FLT1 genes and, optionally, reagents for the determination of the expression levels of one or more housekeeping genes, wherein the reagents adequate for determining the expression levels of the NPR3/C5orf23, CDKN2B and FLT1 genes comprise at least 10% of the probes present in the kit.

In yet another aspect, the invention relates to the use of a kit according to the invention for predicting the outcome of a patient suffering colorectal cancer, or for selecting a patient which is likely to benefit from adjuvant therapy after surgical resection of colorectal cancer.

### BRIEF DESCRIPTION OF THE FIGURES

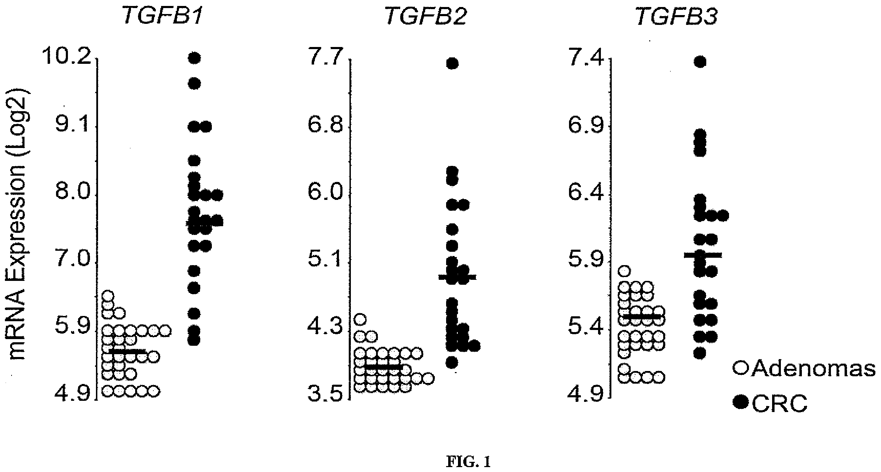

**Figure 1**: TGF-beta signalling increases during adenoma-carcinoma transition during CRC progression. The figure shows the levels of the *TGFB1, 2* and *3* mRNA levels in CRC samples (●) and in adenomas (○).

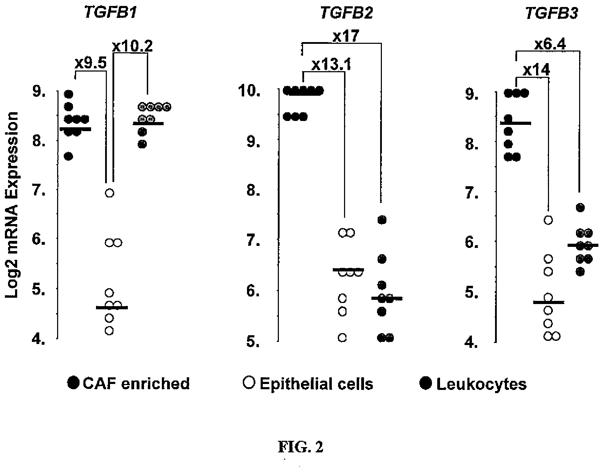

**Figure 2****:** TGF-beta signalling is contributed by CRC associated fibroblasts (CAFs). Freshly resected primary CRC tumours where dissociated and specific tumor cell populations were purified by FACS using a combination of surface markers. CAFs exhibit high relative mRNA levels of *TGFB2* and *TGFB3* compared to epithelial cells and leukocytes. *TGFB1* mRNA levels are comparable between CAFs and Leukocytes, yet higher than those in epithelial cells. Results are obtained by microarray analysis (n=8 CRC patients).

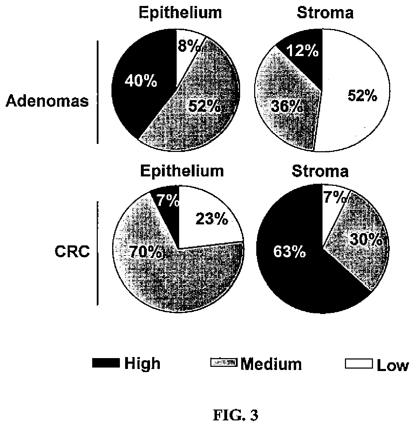

**Figure 3**: TGF-beta signalling acts preferentially over the stromal component of CRC. Classification of Adenoma (n = 25) and CRC samples (n = 30) analyzed according to the distribution and intensity of nuclear p-SMAD3 reactivity in epithelial and stromal cells. Whereas the stroma of most adenomas contained few p-SMAD3 highly positive cells and stained weakly overall, a large proportion of CRCs (63%) were characterized by an abundance of stromal cells with strong nuclear p-SMAD3 staining, indicative of active TGF-beta signalling in these cells.

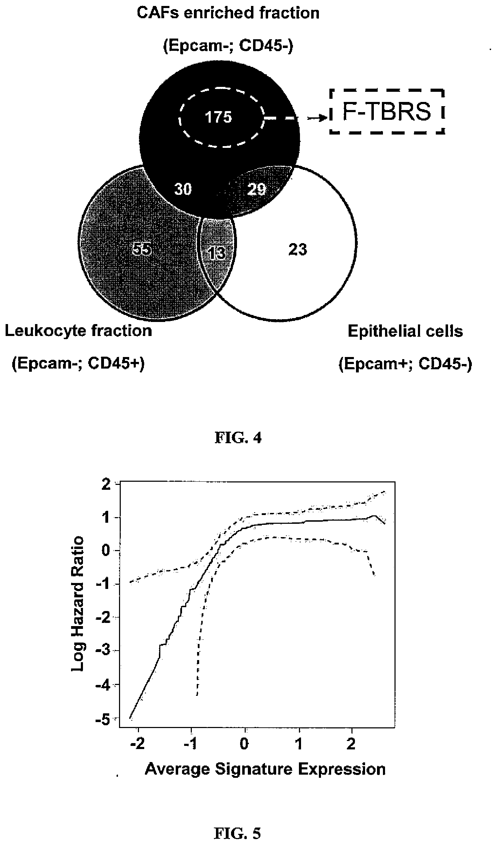

**Figure 4****:** shows how the F-TBRS was derived. TGF-beta induced genes were obtained by microarray analysis of CCD-18Co normal colon fibroblasts in culture treated or not with TGFB. We further refined our classifier by analyzing their differential expression in FACS-purified CRC cell populations from patients (Venn diagram). F-TBRS is composed of those 175 probes specifically upregulated in the CAF-enriched cell population compared with the other two fractions (>2 fold, p<0.05). 65 probes induced by TGF-beta in CCD-18Co were not significantly enriched in any of the three cell populations.
**Figure 5****:** Expression of the F-TBRS signature displays an incremental effect on the risk of recurrence.

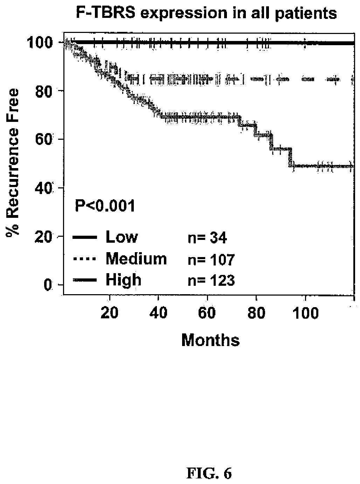

**Figure 6****:** Kaplan-Meier curves show the estimated probability of remaining disease-free upon therapy depending on the average expression level of F-TBRS.

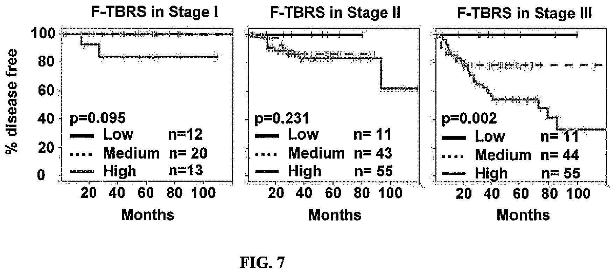

**Figure 7****:** Kaplan-Meier curves show the estimated probability of remaining disease-free upon therapy depending on the average expression level of F-TBRS in patient samples previously grouped according to their AJCC stage. P-values refer to overall differences between the three groups.

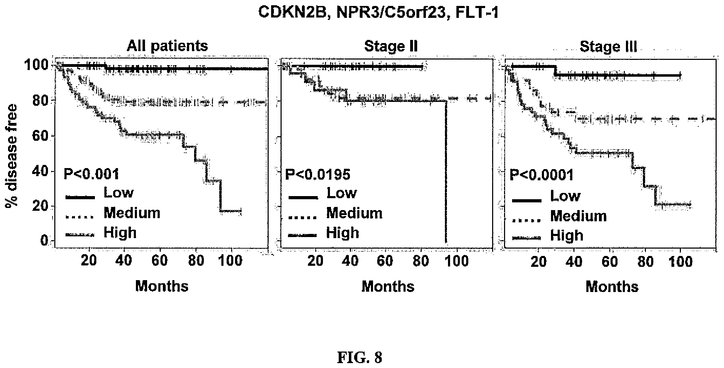

**Figure 8****:** Kaplan Meier curves show survival depending on the average expression of the 3 predictors, CDKN2B, NPR3/C5orf23, FLT-1 for all patients (A), or for stage II (B) or stage III (C) patients.

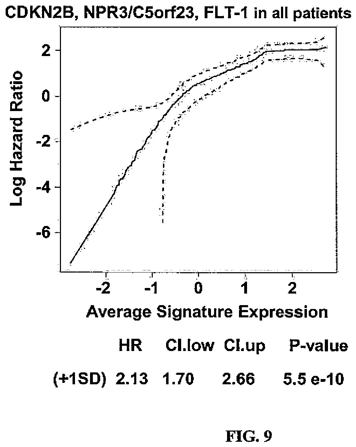

**Figure 9****:** shows incremental and approximately linear correlation between the expression of the 3 predictors, CDKN2B, NPR3/C5orf23, FLT-1 and the risk of recurrence.

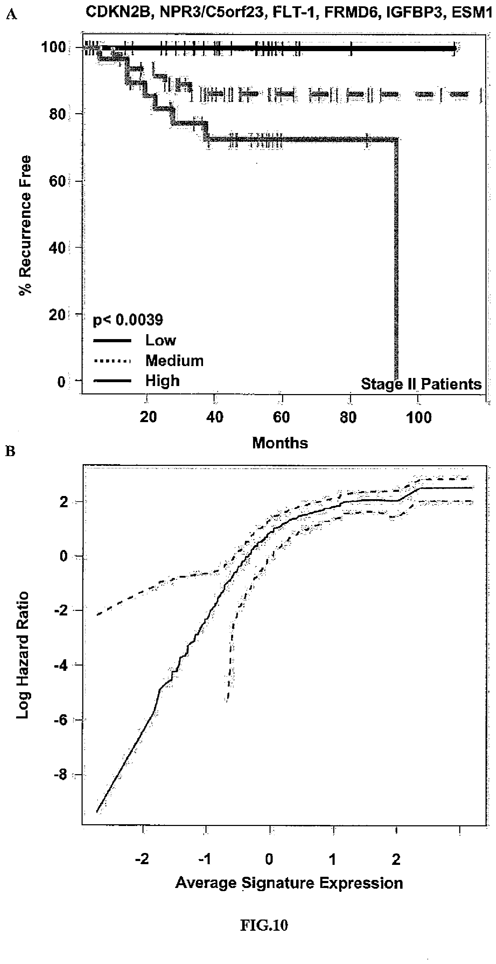

**Figure 10****: A.** Kaplan Meier curves show survival depending on the average expression of the 6 predictors FRMD6, ESM1, IGFBP3, FLT1, NPR3/C5orf23 and CDKN2B for Stage II patients. **B.** Incremental and approximately linear correlation between expression of the 6 predictors FRMD6, ESM1, IGFBP3, FLT1, NPR3/C5orf23 and CDKN2B and the risk of recurrence in all patients.

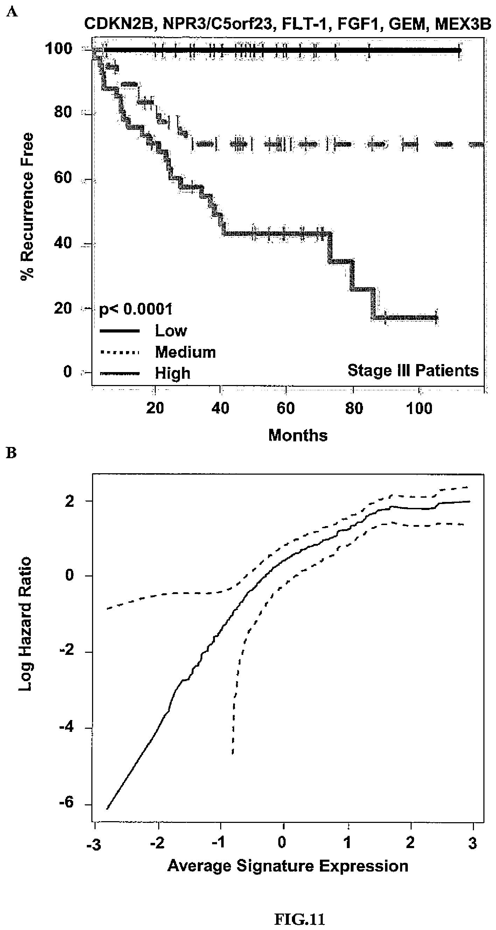

**Figure 11****: A.** Kaplan Meier curves show survival depending on the average expression of the 6 predictors CDKN2B, NPR3/C5orf23, FLT1, GEM, FGF1 and MEX3B in Stage III patients. **B.** Incremental and approximately linear correlation between the expression of the 6 predictors CDKN2B, NPR3/C5orf23, FLT1, GEM, FGF1 and MEX3B and the risk of recurrence in all patients.

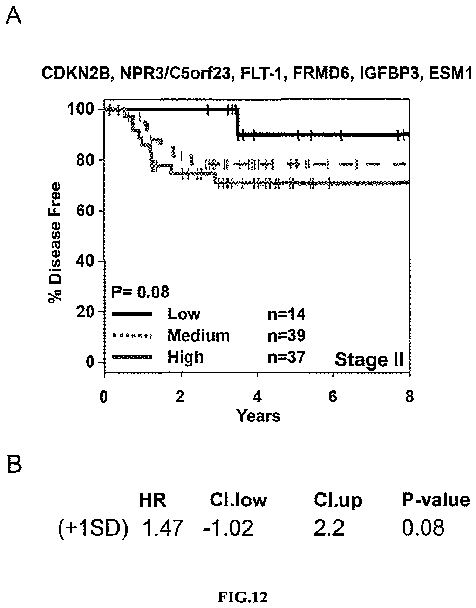

**Figure 12****:** In silico Validation. Colostage II predictor performance in a completely independent set of stage II CRC patients (GSE33113). **A.**- Kaplan Meier curves show probability of remaining disease-free upon therapy depending on the average expression of the Colostage II predictor. **B.** For every increment (+1SD) in the average expression of the colostage II predictor there is a 1.47 increase in the risk to experience recurrence.

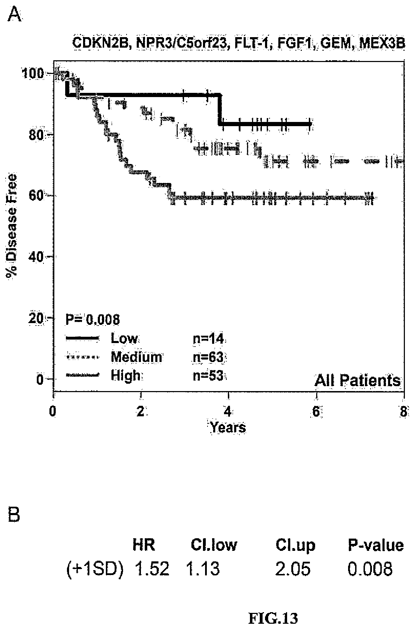

**Figure 13****:** In silico Validation. Colostage III signature performance in a completely independent dataset of stage II and stage III CRC patients (GSE37892). **A.**- Kaplan Meier curves show probability of remaining disease-free upon therapy depending on the average expression of the Colostage III predictor. **B.** For every increment (+1SD) in the average expression of the colostage III predictor there is a 1.52 increase in the risk to experience recurrence.

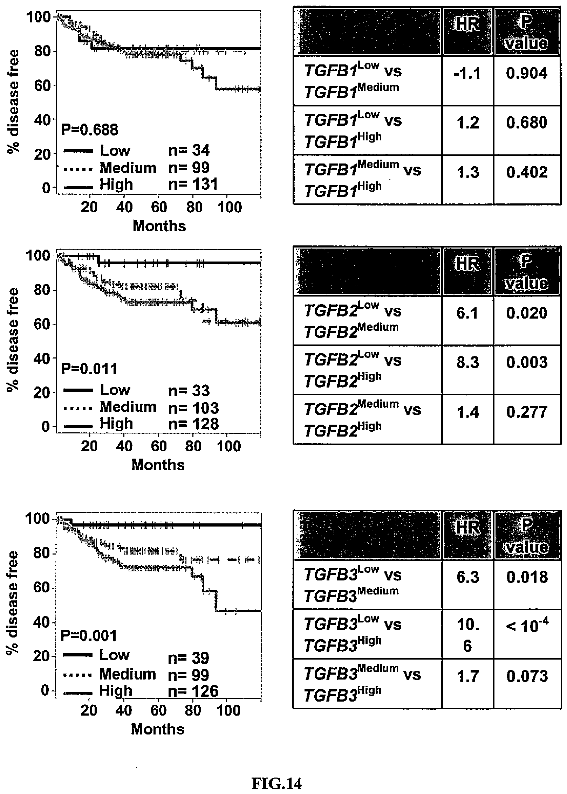

**Figure 14****:** Shows that *TGFB2* and *TGFB3* mRNA levels in tumors predict CRC relapse. A. Kaplan-Meier curves show lower recurrence-free survival over time for patients bearing CRCs with an average high expression (black line) compared to medium (dashed gray) or low (solid grey) expression of *TGFB1, TGFB2* and *TGFB3* mRNA. (Bottom right: overall p-value). B. Hazard Ratios (HR) and p-values for recurrence-free survival probability over time comparing patients bearing low vs. medium, low vs. high and medium vs. high expression of *TGFB1, TGFB2* and *TGFB3. TGFB2* and *TGFB3* expression levels have statistically significant predictive power for disease free survival.

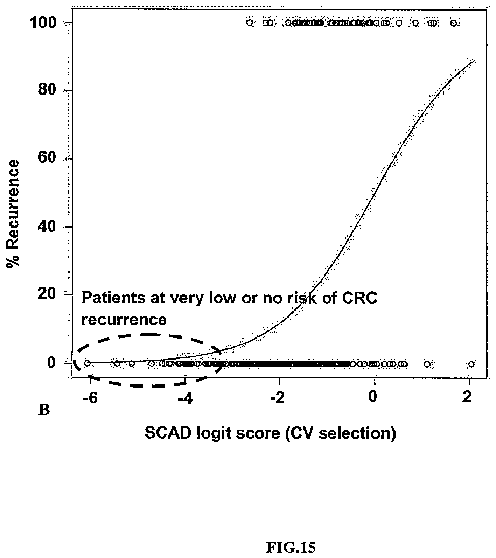

**Figure 15****:** Distribution of CRC patients according to SCAD coefficient according to their TGF-beta 2 and -beta 3 expression levels for all patients.

### DETAILED DESCRIPTION OF THE INVENTION

### Prognostic methods of the invention

### Prognostic methods based on F-TBRS and on the minisignatures comprising 3 or 6 genes

The authors of the present invention have identified a set of genes which provide a reliable method for the identification of CRC patients at greatest and least risk (eg, "high-risk" stage II and "low-risk" stage III colon cancer) of suffering relapse. For instance, as shown in example 2 of the application, a set of 127 genes induced by TGF-beta signaling in normal colon fibroblasts (CCD-co-18) is differentially expressed in cancer associated fibroblasts in response to TGF-beta signaling with respect to epithelial cells and leukocytes purified from colorectal tumours. This set of genes allows predicting relapse of patients with a sensitivity that outperforms AJCC staging. Moreover, by further refining the above signature, the authors of the present invention have selected a small subset of genes from the 127 gene signature that allows predicting the risk of recurrence. Thus, in a first aspect, the invention relates to a method (hereinafter first prognostic method of the invention) for predicting the outcome of a patient suffering colorectal cancer comprising the determination of the expression levels of the NPR3/C5orf23, CDKN2B and FLT1 genes in a sample from said patient wherein an increased expression level of said genes with respect to a reference value for said genes is indicative of an increased likelihood of a negative outcome of the patient or wherein a decreased expression level of said genes with respect to a reference values for said gene is indicative of an increased likelihood of a positive outcome of the patient.

The term "predicting the outcome", is used herein to refer to the likelihood that a patient will have a particular clinical outcome, whether positive or negative. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as chemotherapy. The prediction may include prognostic factors.

As will be understood by those skilled in the art, the prediction, although preferred to be, need not be correct for 100% of the subjects to be diagnosed or evaluated. The term, however, requires that a statistically significant portion of subjects can be identified as having an increased probability of having a given outcome. Whether a subject is statistically significant can be determined without further ado by the person skilled in the art using various well known statistic evaluation tools, e.g., determination of confidence intervals, p-value determination, cross-validated classification rates and the like etc. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.01, 0,005 or lower.

The term "patient", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race.

The term "colorectal cancer" is used in the broadest sense and refers to (1) all stages and all forms of cancer arising from epithelial cells of the large intestine and/or rectum and/or (2) all stages and all forms of cancer affecting the lining of the large intestine and/or rectum. In the staging systems used for classification of colorectal cancer, the colon and rectum are treated as one organ.

In a preferred embodiment, the patient has a stage I, a stage II, a stage III or a stage IV tumor, wherein Stage I is defined as either T1 N0 M0 or T2 N0 M0; Stage II is defined as T3 N0 M0 or T4 N0 M0; Stage III is defined as any T, N1-2; M0 and Stage IV correspond to any T, any N, M1. According to the tumor, node, metastasis (TNM) staging system of the American Joint Committee on Cancer (AJCC) (Greene et al. (eds.), AJCC Cancer Staging Manual. 6th Ed. New York, N.Y.: Springer; 2002), the various stages of colorectal cancer are defined as follows:
- Tumor: T1: tumor invades submucosa; T2: tumor invades muscularis propria; T3: tumor invades through the muscularis propria into the subserose, or into the pericolic or perirectal tissues; T4: tumor directly invades other organs or structures, and/or perforates.
- Node: N0: no regional lymph node metastasis; N1: metastasis in 1 to 3 regional lymph nodes; N2: metastasis in 4 or more regional lymph nodes.
- Metastasis: M0: mp distant metastasis; M1: distant metastasis present.

In a preferred embodiment, the patient the outcome of which is to be predicted is a patient which has been diagnosed with colorectal cancer and which has had surgical resection of the cancer. In a preferred embodiment, the patient has had a surgical resection of a stage I tumor, of a stage II tumor, of a stage III tumor or of a stage IV tumor.

In the present invention, the term "sample" or "biological sample" means biological material isolated from a subject. The biological sample can contain any biological material suitable for detecting the desired biomarker and can comprise cell and/or non-cell material of the subject. The sample can be isolated from any suitable tissue or biological fluid such as for example, prostate tissue, blood, blood plasma, serum, urine, cerebrospinal liquid (CSF) or feces. The samples used for the determination of the marker genes are preferably colorectal tissue samples obtained by biopsy.

Alternatively, the samples are biofluid samples. The terms "biological fluid" and "biofluid" are used interchangeably herein and refer to aqueous fluids of biological origin.

The biofluid may be obtained from any location (such as blood, plasma, serum, urine, bile, cerebrospinal fluid, aqueous or vitreous humor, or any bodily secretion), an exudate (such as fluid obtained from an abscess or any other site of infection or inflammation), or fluid obtained from a joint (such as a normal joint or a joint affected by disease such as rheumatoid arthritis).

In a first step, the first method of the invention comprises the determination of the expression levels of the NPR3/C5orf23, CDKN2B and FLT1 genes in a sample from said patient.

The term "NPR3/C5orf23", as used herein, refers to open reading frame 23 found in chromosome 5, corresponding to NPR3 natriuretic peptide receptor C/guanylate cyclase C (atrionatriuretic peptide receptor C), also known as FLJ14054 or hypothetical protein LOC79614. The human NPR3/C5orf23, gene is depicted under accession number NG_028162.1 in the GenBank database.

The term "CDKN2B", as used herein, refers to cyclin-dependent kinase inhibitor 2B, also known as p15, MTS-22, MTS21, p15 CDK inhibitor, INK4B1, P15, p14_CDK inhibitor, TP15, p15INK4b, CDK inhibitory protein, CDK4I1, p14_INK4B2, multiple tumor suppressor 2, cyclin-dependent kinases 4 and 6 binding protein, p14-INK4b, p15_INK4B2, p15-INK4b or p15INK4B3. The different isoforms of the human CDKN2B mRNA are depicted in the GenBank database under accession numbers NM_078487.2 and NM_004936.3.

The term "FLT1", as used herein, refers to fins-related tyrosine kinase 1 and also known as vascular endothelial growth factor receptor, vascular permeability factor receptor or VEGFR-1. The human gene encoding FLT1 is depicted under accession number NG_012003.1 in the GenBank database.

Moreover, in addition to the determination of the markers mentioned above, the method according to the invention may further comprise the determination of one or more markers selected from the group consisting of FRMD6, IGFBP3, ESM1, FGF1, GEM, MEX3B, WNT2, NGF, MSC, SETBP1, FLJ10357, DACT, MURC and Col10A1, wherein increased expression levels of one or more of said genes with respect to a reference value is indicative that the patient shows increased risk of recurrence or wherein decreased expression levels of one or more of said genes with respect to a reference value for each gene is indicative that the patient shows low risk of recurrence.

The term "FRMD6", as used herein, refers to the FRMD6 domain containing 6, also known as EX1, Willin, C14orf31, MGC17921, c14_5320. The human FRMD6 gene is depicted in the GenBank database under accession number AL079307.7.

The term "IGFBP3", as used herein, refers to the Insulin-like growth factor binding protein 3, also known as IBP3 or BP-53. The human IGFBP3 gene is depicted in the GenBank database under accession number NG_011508.1 (positions 5001 to 14028).

The term "ESM1", as used herein, refers to endothelial cell-specific molecule 1, also known as endocan. The human ESM1 gene is depicted in the GenBank database under accession number NC_000005.9 (complement of positions 54273695 to 54281414.

The term "FGF1", as used herein, refers to fibroblast growth factor 1 (acidic), also known as AFGF, ECGF, FGFA, ECGFA, ECGFB, HBGF1, GLIO703, ECGF-beta or FGF-alpha. The human FGF1 gene is depicted in the GenBank database under accession number NC_000005.9 (complement of positions 141971743 to 142077635).

The term "GEM", as used herein, refers to GTP binding protein overexpressed in skeletal muscle, also known as KIR or MGC26294. The human GEM gene is depicted in the GenBank database under accession number NC_000008.10 (complement of positions 95261481 to 95274547).

The term "MEX3B", as used herein, refers to a RNA-binding protein, also known as RKHD3, MEX-3B, RNF195, MGC117199 or DKFZp434J0617. The human MEX3B gene is depicted in the GenBank database under accession number NC_000015.9 (complement of positions 82334128 to 82338361).

The term "WNT2", as used herein, refers to the wingless-type MMTV integration site family member 2, also known as IRP or INT1L1. The human WNT2 gene is depicted in the GenBank database under accession number NC_000007.13 (complement of positions 116916685 to 116963343).

The term "NGF", as used herein, refers to nerve growth factor, also known as NGFB, HSAN5, Beta-NGF, MGC161426 or MGC161428. The human NGF gene is depicted in the GenBank database under accession number NG_007944.1 (positions 5001 to 57321).

The term "MSC", as used herein, refers to the musculin gene, also known as ABF1, MYOR, ABF-1 or bHLHa22. The human MSC gene is depicted in the GenBank database under accession number NC_000008.10 (complement of positions 72753777 to 72756731).

The term "SETBP1", as used herein, refers to SET binding protein 1, also known as SEB, KIAA0437 or DKFZp666J1210. The human gene encoding SETBP1 is shown in the GenBank database under accession number G_027527.1 (positions 5001 to 393338).

The term "FLJ10357", as used herein, refers to Rho guanine nucleotide exchange factor (GEF) 40, also known as ARHGEF40, SOLO or Protein SOLO. The human gene encoding FLJ10357 is shown in the GenBank database under accession number NC_000014.8 (positions 21538527 to 21558036). The gene FLJ10357 is also known as ARHGEF40.

The term "DACT1", as used herein, refers to dapper, antagonist of beta-catenin, homolog1, also known as DAPPER1, FRODO, DPR1, HDPR1, THYEX3, DAPPER, Hepatocellular carcinoma novel gene 3 protein, HNG3, or hDPR1. The human gene encoding DACT1 is shown in the GenBank database under accession number NC_000014.8 (positions 59104757 to 59115039).

The term "MURC", as used herein, refers to muscle-related coiled-coil protein. The human gene encoding MURC is shown in the GenBank database under accession number NC_000009.11 (positions 103340336 to 103350180).

The term "Col10A1", as used herein, refers to collagen, type X, alpha 1. The human gene encoding Col10A1 is shown in the GenBank database under accession number NG_008032.1 (positions 5001 to 12212).

It will be understood that the method according to the present invention may comprise the determination of any naturally occurring polymorphic variant of one or more of the above genes.

In a preferred embodiment, the marker genes used in the first method of the invention are FRMD6, ESM1, IGFBP3, FLT1, NPR3/C5orf23 and CDKN2B and the patient is a stage II CRC patient.

In a preferred embodiment, the marker genes used in the first method of the invention are CDKN2B, NPR3/C5orf23, FLT1, GEM, FGF1 and MEX3B and the patient is a stage III CRC patient.

In a preferred embodiment, the method of the invention comprises the determination of the expression levels of the genes FRMD6, IGFBP3, ESM1, FGF1, GEM, MEX3B, WNT2, NGF, MSC, NPR3/C5orf23, CDKN2B, SETBP1, FLJ10357, DACT, MURC, FLT1 and Col10A1.

The method according to the present invention may further comprise the determination of the expression levels of one or more of the genes forming the F-TBRS, i.e. genes which are differentially expressed between the cell population enriched in cancer associated fibroblasts (enriched CAFs; EPCAM- CD45-) and the EPCAM+ and the EPCAM- cd45+ cell populations and having at least a 2-fold increase in the first cell population with respect to the second and third cell populations wherein increased expression levels of said genes with respect to reference values for said genes is indicative that the patient shows increased risk of recurrence or wherein decreased expression levels of said genes with respect to reference values for said gene is indicative that the patient shows low risk of recurrence.

In a preferred embodiment, the method for predicting the outcome of a patient suffering colorectal cancer comprises the determination of the expression levels of the genes ANGPTL2, ANGPTL4, APBB2, BMPR2, BPGM, C13orf33, C5orf13, NPR3/C5orf23, CACHD1, CALD1, CDH6, CDKN2B, CILP, CNTN1, COL10A1, COL12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A and ZEB1 genes and the genes which hybridize specifically with the probes having the sequences SEQ ID NO:1 to 13, wherein increased expression levels of said genes with respect to reference values for said genes is indicative of an increased likelihood of a negative outcome of the patient or wherein decreased expression levels of said genes with respect to reference values for said gene is indicative of an increased likelihood of a positive outcome of the patient.

The term "specifically hybridizing", as used herein, refers to conditions which allow the hybridization of two polynucleotide sequences under high stringent conditions or moderately stringent conditions. The expressions "high stringent conditions" and "moderately stringent conditions" are defined below in respect to the kit of the invention and are equally applicable in the context of the present method.

Virtually any conventional method can be used within the frame of the invention to detect and quantify the levels of said marker genes. By way of a non-limiting illustration, the expression levels are determined by means of the quantification of the levels of mRNA encoded by said genes or by means of the quantification of the protein levels.

Methods for determining the quantity of mRNA are well known in the art. For example the nucleic acid contained in the sample (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Preferably quantitative or semi-quantitative RT-PCR is preferred. Real-time quantitative or semi-quantitative RT-PCR is particularly advantageous. Preferably, primer pairs were designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA). Preferably, the quantity of mRNA is measured by quantitative or semi-quantitative RT-PCR or by real-time quantitative or semi-quantitative RT-PCR.

Alternatively, it is also possible to determine the expression levels of the marker genes by means of the determination of the expression levels of the proteins encoded by said genes, since if the expression of genes is increased, an increase of the amount of corresponding protein should occur. The determination of the expression levels of the different proteins can be carried out using any conventional method. By way of a non-limiting example, said determination can be carried out using antibodies with the capacity for binding specifically to the protein to be determined (or to fragments thereof containing the antigenic determinants) and subsequent quantification of the resulting antigen-antibody complexes. The antibodies that are going to be used in this type of assay can be, for example polyclonal sera, hybridoma supernatants or monoclonal antibodies, antibody fragments, Fv, Fab, Fab' and F(ab')2, scFv, diabodies, triabodies, tetrabodies and humanized antibodies. At the same time, the antibodies may or may not be labeled. Illustrative, but non-exclusive, examples of markers that can be used include radioactive isotopes, enzymes, fluorophores, chemoluminescent reagents, enzyme cofactors or substrates, enzyme inhibitors, particles, dyes, etc. There is a wide variety of well known assays that can be used in the present invention, using non-labeled antibodies (primary antibody) and labeled antibodies (secondary antibodies); these techniques include Western-blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of biochips or protein microarrays including specific antibodies or assays based on the colloidal precipitation in formats such as reagent strips. Other forms of detecting and quantifying the protein include affinity chromatography techniques, ligand-binding assays, etc.

Once the expression levels of the above genes in a sample from a patient have been determined, the levels are then compared with reference values for each of said gene. Typically, reference values are the expression level of the genes being compared in a reference sample.

A "reference sample", as used herein, means a sample obtained from a pool of healthy subjects which does not have a disease state or particular phenotype. For example, the reference sample may comprise samples from colon mucosa from patients which do not suffer colon cancer or which do not have a history of colon cancer. Alternatively, the reference sample could be a sample or a pool of samples of colon cancer with a low risk of recurrence. This sample or pool of samples can be obtained from patients which have had surgical resection of the tumor and which have not suffered relapse, preferably in the absence of adjuvant chemotherapy. In another embodiment, the reference sample is a sample from a type I CRC or a pool of type I CRCs.

The suitable reference expression levels of genes can be determined by measuring the expression levels of said genes in several suitable subjects, and such reference levels can be adjusted to specific subject populations (for example, a reference level can be linked to the age so that comparisons can be made between expression levels in samples of subjects of a certain age and reference levels for a particular disease state, phenotype, or lack thereof in a certain age group). In a preferred embodiment, the reference sample is obtained from several healthy subjects or from subjects without prior history of colorectal cancer. Alternatively, the reference sample is a sample or a pool of samples of colon cancer from patients which have had surgical resection of the tumor and which have not suffered relapse, preferably in the absence of adjuvant chemotherapy. The person skilled in the art will appreciate that the type of reference sample can vary depending on the specific method to be performed. Thus, in the case that a diagnosis or prognosis of the disease is to be carried out, the references sample may be a pool of non-tumor colorectal tissue samples, either from individuals that do not have a history of colorectal cancer or from a pool of distal non-tumor tissues with respect to the respective tumor tissues, or a sample or a pool of samples of colon cancer from patients which have had surgical resection of the tumor and which have not suffered relapse, preferably in the absence of adjuvant chemotherapy. In the event that the method of the invention is aimed at determining the effect of a therapy in a patient, the reference sample is preferably a sample obtained from said patient before starting the treatment.

The expression profile of the genes in the reference sample can preferably, be generated from a population of two or more individuals. The population, for example, can comprise 3, 4, 5, 10, 15, 20, 30, 40, 50 or more individuals. Furthermore, the expression profile of the genes in the reference sample and in the sample of the individual that is going to be diagnosed according to the methods of the present invention can be generated from the same individual, provided that the profiles to be assayed and the reference profile are generated from biological samples taken at different times and are compared to one another. For example, a sample of an individual can be obtained at the beginning of a study period. A reference biomarker profile from this sample can then be compared with the biomarker profiles generated from subsequent samples of the same individual. In a preferred embodiment, the reference sample is a pool of samples from several individuals and corresponds to portions of colorectal tissue that are far from the tumor area and which have preferably been obtained in the same biopsy but which do not have any anatomopathologic characteristic of tumor tissue.

Once the expression levels of the marker genes in relation to reference values for said genes have been determined, it is necessary to identify if there are alterations in the expression of said genes (increase or decrease of the expression). The expression of a gene is considered increased in a sample of the subject under study when the levels increase with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Similarly, the expression of a gene is considered decreased when its levels decrease with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

Lastly, the patient is then classified as having a high risk of negative outcome if the marker genes show increased expression levels with respect to a reference sample and as having a low risk of negative outcome if the marker genes show decreased expression levels with respect to a reference sample. In a preferred embodiment, a patient is then classified as having a high risk of negative outcome if the expression levels of the gene is higher than the expression level of the same gene in a sample or in a pool of samples of colon cancer from patients which have had surgical resection of the tumor and which have not suffered relapse, preferably in the absence of adjuvant chemotherapy.

The term "positive outcome" in relation to CRC means an improvement in any measure of patient status, including those measures ordinarily used in the art, such as an increase in the duration of Recurrence-Free interval (RFI), an increase in the time of Overall Survival (OS), an increase in the time of Disease-Free Survival (DFS), an increase in the duration of Distant Recurrence-Free Interval (DRFI), and the like. An increase in the likelihood of positive clinical outcome corresponds to a decrease in the likelihood of cancer recurrence.

The term "negative outcome" in relation to CRC means the worsening in any measure of patient status, including those measures ordinarily used in the art, such as a decrease in the duration of Recurrence-Free interval (RFI), a decrease in the time of Overall Survival (OS), a decrease in the time of Disease-Free Survival (DFS), a decrease in the duration of Distant Recurrence-Free Interval (DRFI), and the like. An increase in the likelihood of negative clinical outcome corresponds to an increase in the likelihood of cancer recurrence.

In a preferred embodiment, the outcome in a given patient is measured as the risk of metastasis or as the risk of recurrence.

The term "risk of metastasis", as used herein, refers to a likelihood or probability assessment regarding the chances or the probability that a subject or individual may develop a similar or the same neoplastic disease at an anatomically distant location within a defined time interval, comparable to the one that the subject or individual has been treated for or diagnosed for.

The term "metastasis" as used herein refers to the growth of a cancerous tumor in an organ or body part, which is not directly connected to the organ of the original cancerous tumor. Metastasis will be understood to include micrometastasis, which is the presence of an undetectable amount of cancerous cells in an organ or body part which is not directly connected to the organ of the original cancerous tumor. In a preferred embodiment, the metastasis is liver metastasis.

The term "risk of recurrence", as used herein, refers to a likelihood or probability assessment regarding the chances or the probability that a subject or individual may be afflicted with or may be developing a similar or the same neoplastic disease (either at the same anatomical location or an event at an anatomically distant location), within a defined time interval, comparable to the one that the subject or individual has been treated for or diagnosed for.

The method according to the invention further contemplates the possibility of predicting the outcome of a patient combining the expression levels of the different marker genes mentioned above with one or more clinical prognostic factors.

Prognostic factors are those variables related to the natural history of colorectal cancer, which influence the recurrence rates and outcome of patients once they have developed colorectal cancer. Clinical parameters that have been associated with a worse prognosis include, for example, lymph node involvement, and high grade tumors. Prognostic factors are frequently used to categorize patients into subgroups with different baseline relapse risks. In a preferred embodiment, the clinical prognostic factor used in the method of the invention is tumor stage, wherein increased tumor stage is indicative of an increased risk of recurrence or wherein decreased tumor stage is indicative that the patient shows low risk of recurrence.

In a preferred embodiment, the clinical prognostic factor is the tumor stage according to the AJCC classification (See for example AJCC Cancer Staging Manual, Seventh Edition (2010) published by Springer- Verlag New York, herein incorporated by reference) and as defined above. The term "tumor stage", as mentioned above, is a value that is determined on the basis of the TNM value for the tumor. Thus, the stage I corresponds to T1 N0 M0 or T2 N0 M0; Stage II correspond to T3 N0 M0 or T4 N0 M0; Stage III corresponds to any T, N1-2; M0 and Stage IV corresponds to any T, any N and M1.

Thus, in a preferred embodiment, the invention further comprises the determination of the tumor stage in the patient wherein a high tumor stage is indicative of an increased risk of recurrence or wherein a low tumor stage is indicative of a decreased risk of recurrence.

The term "low tumor stage", as used herein, refers to an AJCC stage of I or II.

The term "high tumor stage", as used herein, refers to an AJCC stage of III or IV.

Patients analysed according to the present invention may or may not have been treated with one or more therapies aimed at decreasing tumor size prior to the determination. Thus, in a preferred embodiment, the patients have not been treated prior to the determination of the of the expression levels of the different genes according to the invention. In another embodiment, the patients are treated prior to the determination of the expression levels of the different genes according to the invention with a therapy selected from the group consisting of chemotherapy, radiotherapy or surgery.

The terms "chemotherapy", "radiotherapy" and "surgery" are defined in detailed below and are used with the same meaning in the context of the present invention.

### Prognostic methods based on the expression levels of TGF-beta2 and TGF-beta3 (not part of the invention)

The authors of the present invention have also observed that the expression levels of TGF-beta2 and/or TGF-beta3 are also good predictors of the outcome of patients suffering from CRC. Given the fact that TGF-beta2 and/or TGFbeta3 are secreted molecules, this finding allows the determination of the prognosis of a patient by determining the expression levels of TGF-beta2 and/or TGF-beta3 in biofluids, providing non-invasive means for the predictive method according to the invention.

Thus, also disclosed is a method (hereinafter the second prognostic method) for predicting the outcome of a patient suffering colorectal cancer comprising the determination in a sample from said patient of the expression levels of the TGF-beta2 and/or TGF-beta3 genes wherein increased expression levels of said gene or genes with respect to a reference value for each gene is indicative of an increased likelihood of a negative outcome or wherein decreased expression levels of said gene or genes with respect to a reference value for each gene is indicative of an increased likelihood of a positive outcome.

The terms and expressions "predicting the outcome", "colorectal cancer", "sample", "patient", "increased expression levels", "decreased expression levels", "reference value", "positive outcome" and "negative outcome" have been described in detail in the context of the first prognostic method of the invention and are used with the same meaning in the context of the present method.

In a preferred disclosure, the patient which outcome is to be predicted is a patient which has been diagnosed with colorectal cancer and which has had surgical resection of the cancer.

In a first step, the second prognostic method comprises the determination in a sample from the patient of the expression levels of the TGF-beta2 and/or TGF-beta3 genes.

The term "TGFbeta2", as used herein, refers to the transforming growth factor beta 2, as shown in the NBCI database under accession numbers NP_001129071 (isoform 1) or NP_003229 (isoform 2) for the human orthologs, CAB42003 for the rat orthologs, and AAH11170 for the mouse ortholog. The term "TGFbeta2" also refers to naturally occurring variants and polymorphic forms of any of the above sequences.

The term "TGFbeta3", as used herein, refers to transforming growth factor (TGF) beta 3 corresponding to amino acids 301 to 412 of the human TGF-beta3 preproprotein ortholog as shown under accession number NP_003230 in the NCBI database, to amino acids 301 to 412 of the rat TGF-beta3 preproprotein ortholog as shown under accession number NP_037306. The term "TGFbeta3" also refers to naturally occurring variants and polymorphic forms of any of the above sequences.

The determination of the expression levels of the TGF-beta2 and/or TGF-beta3 genes can be carried out by determining the mRNA levels for said genes or by determining the levels of the proteins encoded by said genes. Suitable procedures for determining the expression levels of a given mRNA or polypeptide have been described in detail above in the context of the first prognostic method of the invention.

In yet another disclosure, the method is carried out in a sample selected from the group consisting of a tumor biopsy or a biofluid. In another disclosure, when the sample is a biofluid, the biofluid is selected from the group consisting of blood, plasma and serum.

In a preferred disclosure, the determination of the expression levels of TGF-beta2 and/or of TGF-beta3 is carried out by RT-PCR. In yet another disclosure, wherein the sample is a tumor sample, then the expression levels of TGF-beta2 and/or TGFbeta3 is carried out by RT-PCR. In another disclosure, wherein the sample is a biofluid, then the expression levels are determined by measuring the levels of the corresponding TGF-beta2 and TGF-beta3 polypeptides.

The method further comprises, in addition to the determination of the expression levels of the above genes, the determination of the tumor stage wherein a high tumor stage is indicative of an increased risk of recurrence or wherein a low tumor stage is indicative that the patient shows low risk of recurrence. The terms "high tumor stage" and "low tumor stage" have been defined in detail above and is used with the same meaning in the context of the present method.

In a preferred disclosure, the prognostic method is carried out in a sample from a patient suffering colorectal cancer wherein the colorectal cancer is a stage II or stage III colorectal tumor. In another disclosure, the method is carried out in a patient which has had surgical resection of the tumor.

In yet another disclosure, the determination of the outcome in a patient according to the present method is carried out by determining the risk of metastasis at the moment of diagnosis or as the risk of recurrence.

### Personalized therapeutic methods according to the invention

The prognostic methods defined above also allow providing personalized therapies to patients suffering colorectal cancer. In particular, patients which are considered as having a high risk of relapse will most likely benefit from an additional therapy after surgery. Conversely, patients showing low risk of relapse may forego additional therapeutic treatment following surgery.

### Personalised medicine based on expression levels of the F-TBRS and the minisignatures

Thus, further disclosed is a method (hereinafter first personalized therapeutic method) for selecting a suitable treatment for colorectal cancer in a patient comprising the determination of the expression levels of the NPR3/C5orf23, CDKN2B and FLT1 genes in a sample from said patient, wherein an increased expression level of said genes with respect to a reference value for said genes is indicative that the patient is candidate for receiving radiotherapy or chemotherapy after surgical treatment or wherein a decreased expression level of said genes with respect to a reference values for said gene is indicative that the patient is not candidate for receiving radiotherapy or chemotherapy after surgical treatment.

As used herein, "treatment" refers to clinical intervention in an attempt to prevent, cure, delay, reduce the severity of, or ameliorate one or more symptoms of the disease or disorder or recurring disease or disorder, or in order to prolong the survival of a patient beyond that expected in the absence of such treatment.

The term "colorectal cancer" has been described in detail in the context of the prognostic methods of the invention and is used with the same meaning in the context of the personalized methods according to the invention.

In a first step, the first personalized therapeutic method comprises the determination of the expression level of the NPR3/C5orf23, CDKN2B and FLT1 genes in a sample from said patient.

The terms "colorectal cancer", "patient", "NPR3/C5orf23 gene", "CDKN2B gene", "FLT1gene" "expression levels", "sample" have been described in detail above and are equally applied to the methods according to the present method.

Moreover, in addition to the determination of the markers mentioned above, the first personalized therapeutic method may further comprise the determination of one or more markers selected from the group consisting of FRMD6, IGFBP3, ESM1, FGF1, GEM, MEX3B, WNT2, NGF, MSC, SETBP1, FLJ10357, DACT, MURC and Col10A1, wherein increased expression levels of one or more of said genes with respect to a reference value is indicative that the patient is candidate for receiving therapy after surgical treatment or wherein a decreased expression level of said genes with respect to a reference values for said gene is indicative that the patient is not candidate for receiving therapy after surgical treatment.

In a preferred disclosure, the first personalized therapeutic method comprises the determination of the expression levels of genes CDKN2B, NPR3/C5orf23, FLT1, FRMD6, IGFBP3 and ESM1 and the determination is carried out in a sample from a patient suffering from stage II colorectal cancer.

In another preferred disclosure, the first personalized therapeutic method comprises the determination levels of the CDKN2B, NPR3/C5orf23, FLT1, FGF1, GEM, and MEX3B genes and said determination is carried out in a patient suffering from stage III colorectal cancer.

In yet another disclosure, the first personalized therapeutic method further comprises the determination of the expression levels of one or more genes shown in Table 1, which are characterized in that they have a FC value higher than 2 in the CAFs enriched vs. EPCAM+ column wherein increased expression levels of said genes with respect to reference values for said genes is indicative that the patient is candidate for receiving adjuvant therapy after surgical treatment or wherein decreased expression levels of said genes with respect to reference values for said genes is indicative that the patient is not a candidate for receiving therapy after surgical treatment.

In a preferred disclosure, the first personalized therapeutic method comprises the determination of the expression levels of the genes ANGPTL2, ANGPTL4, APBB2, BMPR2, BPGM, C13orf33, C5orf13, NPR3/C5orf23, CACHD1, CALD1, CDH6, CDKN2B, CILP, CNTN1, COL10A1, COL12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A and ZEB1 genes and to the genes which hybridize specifically with the probes having the sequences SEQ ID NO:1 to 13, wherein increased expression levels of said genes with respect to reference values for said genes is indicative that the patient is candidate for receiving adjuvant radiotherapy or chemotherapy after surgical treatment wherein decreased expression levels of said genes with respect to a reference value for one or more of said genes is indicative that the patient is not a candidate for receiving radiotherapy or chemotherapy after surgical treatment.

The term "specifically hybridizing", as used herein, refers to conditions which allow the hybridization of two polynucleotide sequences under high stringent conditions or moderately stringent conditions. The expressions "high stringent conditions" and "moderately stringent conditions" are defined below in respect to the kit of the invention and are equally applicable in the context of the present method.

The expression levels of the different genes used in the first personalized therapeutic method can be determined by determining the levels of the mRNA encoded by said genes or by determining the levels of the polypeptide encoded by said genes.

In a second step, the first personalized therapeutic method comprises the identification of those patients showing increased expression levels of said genes with respect to a reference value for said genes as candidates for receiving adjuvant radiotherapy or chemotherapy after surgical treatment or of those patients showing decreased expression levels of the gene with respect to a reference value as a patient which is not a candidate for receiving radiotherapy or chemotherapy after surgery.

The term "surgery", as used herein, means any therapeutic procedure that involves methodical action of the hand or of the hand with an instrument, on the body of a human or other mammal, to produce a curative or remedial.

As used herein the term "chemotherapy", "chemotherapeutic drug" refers broadly to the use of a chemical drug or a combination thereof for the treatment of cancer, tumors or malign neoplasia, including both cytotoxic or cytostatic drugs. Examples of chemotherapy agents which may be in accordance to the present invention include:
- alkylating agents (for example mechlorethamine, chlorambucil, cyclophosphamide, ifosfamide, streptozocin, carmustine, lomustine, melphalan, busulfan, dacarbazine, temozolomide, thiotepa or altretamine);
- platinum drugs (for example cisplatin, carboplatin or oxaliplatin);
- antimetabolite drugs (for example 5-fluorouracil, capecitabine, 6-mercaptopurine, methotrexate, gemcitabine, cytarabine, fludarabine or pemetrexed);
- anti-tumor antibiotics (for example daunorubicin, doxorubicin, epirubicin, idarubicin, actinomycin-D, bleomycin, mitomycin-C or mitoxantrone);
- mitotic inhibitors (for example paclitaxel, docetaxel, ixabepilone, vinblastine, vincristine, vinorelbine, vindesine or estramustine); and
- topoisomerase inhibitors (for example etoposide, teniposide, topotecan, irinotecan, diflomotecan or elomotecan).

The term "radiotherapy" is a term commonly used in the art to refer to multiple types of radiation therapy including internal and external radiation therapies or radio immunotherapy, and the use of various types of radiations including X-rays, gamma rays, alpha particles, beta particles, photons, electrons, neutrons, radioisotopes, and other forms of ionizing radiations.

In a preferred embodiment, the therapy is neoadjuvant or adjuvant chemotherapy.

The term "neoadjuvant therapy", as used herein, refers to any type of treatment of cancer given prior to surgical resection of the primary tumor, in a patient affected with a cancer. The most common reason for neoadjuvant therapy is to reduce the size of the tumor so as to facilitate a more effective surgery. Neoadjuvant therapies comprise radiotherapy and therapy, preferably systemic therapy, such as hormone therapy, chemotherapy, immunotherapy and monoclonal antibody therapy.

The term "adjuvant therapy", as used herein, refers to any type of treatment of cancer (e.g., chemotherapy or radiotherapy) given as additional treatment, usually after surgical resection of the primary tumor, in a patient affected with a cancer that is at risk of metastasizing and/or likely to recur. The aim of such an adjuvant treatment is to improve the prognosis. Adjuvant therapies comprise radiotherapy and therapy, preferably systemic therapy, such as hormone therapy, chemotherapy, immunotherapy and monoclonal antibody therapy.

In a preferred embodiment, the chemotherapy comprises the use of one or more TGF-beta inhibitors.

In the present invention "a TGFβ inhibitor" is understood as any compound capable of preventing signal transmission caused by the interaction between TGFβ and its receptor. TGFβ1 inhibitors that can be used according to the present invention include compounds preventing the competitive or allosteric binding of TGFβ to its receptor, compounds binding to TGFβ and compounds inhibiting the intracellular signalling of TGFβ. Proper assays to determine the inhibitory capacity of a TGFβ inhibitor include the *in vitro* inhibition of TGFβ biological activity by using the inhibitor in Mv-1-Lu cell proliferation assays as well as the *in vivo* inhibition of TGFβ biological activity by the inhibitor using a model of acute liver damage induced by CC14 (disclosed in WO200519244). For more details about TGF-beta antagonists see also Wojtowicz-Praga (2003).

Suitable TGFβ inhibitors for use in the present invention include, without limitation,
- Soluble proteins which naturally bind to and inhibit TGFβ (LAP, decorin, fibromodulin, lumican, endoglin, α2-macroglobulin).
- Receptors competing with the TGFβ endogenous receptor for binding to the ligand, as BAMBI. In the present invention, a TGFβ soluble receptor is understood as the extracellular domain of TGFβ receptor, which can be obtained physiologically by proteolytic processing of endoglin or betaglycan (type III receptors), or by recombinant technology by expressing only the extracellular domain of type I and type II TGFβ receptors.
- Inhibitory anti-TGFbeta antibodies including, without limitation, multispecific, polyclonal, monoclonal antibodies and F(ab')₂, Fab fragments thereof, such as those described in EP117544, Ling et al., (J. Amer. Soc. Nephrol. 14: 377-388 (2003)), McCormick et al (J. Immunol., 1999, 163:5693-5699) and Cordeiro, (Curr. Opin. Mol. Ther., 2003, 5:199-203);
- Monoclonal and polyclonal antibodies specific to TGFβ receptor and TGFβ receptor soluble forms.
- TGF- beta receptor type I kinase inhibitors as described in, e.g., DaCosta Bayfield, (Mol. Pharmacol., 2004, 65:744-52), Laping, (Curr. Opin. Pharmacol., 2003, 3:204-8) and Laping (Mol. Pharmacol., 2002, 62:58-64)
- Small molecules, e.g. tranilast (N-[3,4-dimethoxycinnamoyl]- anthranilic acid) (Wilkenson, K. A. 2000), SB-431542 (inhibitor of TGF- beta receptor II); 4-(5-Benzol[1,3]dioxol-5-yl-4-pyrldin-2-yl-1H-imidazol-2-yl)-benzamide hydrate, 4-[4-(3,4-Methylenedioxyphenyl)-5-(2-pyridyl)-1H-imidazol-2-yl]-benzamide hydrate, 4-[4-(1,3-Benzodioxol-5-yl)-5-(2-pyridinyl)-1H-imidazol-2-yl]-benzamide hydrate); NPC-30345 (inhibitor of TGF- beta receptor I); LY364947 (inhibitor of TGF- beta receptor I; 4-[3-(2-Pyridinyl)- IH- pyrazol-4-yl]-quinoline); A-83-01 (inhibitor of TGF- beta receptor type I; 3-(6-Methylpyridin-2-yl)-1-phenylthiocarbamoyl-4-quinolin-4-ylpyrazole); LY2157299 (inhibitor of TGF- beta receptor type I; Lilly Research); LY550410 (inhibitor of TGF- beta receptor type I; Lilly Research); LY580276 (inhibitor of TGF- beta receptor type I; Lilly Research); LY566578 (inhibitor of TGF- beta receptor type I; Lilly Research); SB-505124 (selective inhibitor of TGF- beta receptor type I; 2-(5- benzo[1,3]dioxol-5-yl-2-tert-butyl-3H-imidazol-4-yl)-6-methylpyridine hydrochloride); SD-093 (inhibitor of TGF- beta receptor type I; Scios Inc); or SD-208 (inhibitor of TGF- beta receptor type I).
- Peptides being part of TGF-β1, TGF-β2 or TGF-β3 as published in Mittl (1996).
- Peptides comprising the 112 amino acids counted from the end of the TGF-beta 1, TGF-beta 2 or TGF-beta 3 peptide. The start of those peptides is after the RXXR motif, ending 113 amino acids before the end of the TGF-β 1, TGF- β 2 or TGF-β 3 peptide, in which R is the amino acid Arginine and XX represents any amino acid or is even no amino acid.
- TGF-beta specific antisense olgonucleotides, siRNAs or shRNAs.

Other suitable TGF-β inhibitors are those defined in Table 2 as given in Kelly and Morris (J. Immunotoxicol., 2010, 7: 15-26).

| Agent | Target | Phase | Reference |
|---|---|---|---|
| *Antibody* | | | |
| 2G7 | TGF-β1, β2, β3 | Pre-clinical | Arteaga et al., 1993 |
| 1D11 | TGF-β1, β2, β3 | Pre-clinical | Dasch et al., 1989 |
| GC1008 | TGF-β1, β2, β3 | Phase I | Morris et al., 2008 |

| *Antisense oligonucleotides* | | | |
|---|---|---|---|
| AP11014 | TGF-β1 mRNA | Pre-clinical | Schlingensiepen et al., 2004 |
| AP12009 | TGF-β2 mRNA | Phase II/III | Hau et al., 2007 |

| *Peptide aptamers* | | | |
|---|---|---|---|
| Trx-xFoxH1b | Smad2-4 | Pre-clinical | Cui et al., 2005 |
| Trx-SARA | Smad3-4 | Pre-clinical | Zhao and Hoffman, 2006 |

| *Receptor kinase inhibitors* | | | |
|---|---|---|---|
| Ki 26894 | TβRI | Pre-clinical | Ehata et al., 2007 |
| IN-1130 | TβRI | Pre-clinical | Lee et al., 2008 |
| LY2109761 | TβRI/II | Pre-clinical | Melisi et al., 2008 |
| LY364947 | TβRI | Pre-clinical | Peng et al., 2005 |
| LY550410 | TβRI | Pre-clinical | Sawyer et al., 2004 |
| LY580276 | TβRI | Pre-clinical | Sawyer et al., 2004 |
| SB-431542 | TβRI | Pre-clinical | Halder et al., 2005 |
| SB-505124 | TβRI | Pre-clinical | DaCosta-Ryfield et al., 2004 |
| SD-093 | TβRI | Pre-clinical | Subramanian et al., 2004 |
| SD-208 | TβRI | Pre-clinical | Uhl et al., 2004 |
| Sm16 | TβRI | Pre-clinical | Suzuki et al., 2007 |

| *Soluble TGF-β receptors* | | | |
|---|---|---|---|
| Soluble TGFβRII:F | TGF-β | Pre-clinical | Won et al., 1999 |
| Soluble TGFβRIII (betaglycan) | TGF-β | Pre-clinical | Bandyopadhyay et al., 2002 |
| *Dominant-negative TGF-β receptor* | TGF-β | Pre-clinical | Bollard et al., 2002 |
| *Combined vaccine*/*antisense* | TGF-β2 | Phase I/II/III | Nemunaitis et al., 2006; |
| Belagenpumatucel-L (Lucanix™) | | | 2009 |

In a preferred embodiment, the therapy is neoadjuvant or adjuvant chemotherapy.

The term "neoadjuvant therapy", as used herein, refers to any type of treatment of cancer given prior to surgical resection of the primary tumor, in a patient affected with a cancer. The most common reason for neoadjuvant therapy is to reduce the size of the tumor so as to facilitate a more effective surgery. Neoadjuvant therapies comprise radiotherapy and therapy, preferably systemic therapy, such as hormone therapy, chemotherapy, immunotherapy and monoclonal antibody therapy.

The term "adjuvant therapy", as used herein, refers to any type of treatment of cancer (e.g., chemotherapy or radiotherapy) given as additional treatment, usually after surgical resection of the primary tumor, in a patient affected with a cancer that is at risk of metastasizing and/or likely to recur. The aim of such an adjuvant treatment is to improve the prognosis. Adjuvant therapies comprise radiotherapy and therapy, preferably systemic therapy, such as hormone therapy, chemotherapy, immunotherapy and monoclonal antibody therapy.

In another aspect, the invention relates to a method (hereinafter "second personalized therapeutic method of the invention") for selecting a patient which is likely to benefit from adjuvant therapy after surgical resection of colorectal cancer comprising the determination of the expression levels of the NPR3/C5orf23, CDKN2B and FLT1 in a sample from said patient, wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment.

As used herein, the terms "treatment" or "therapy" can be used indistinctly and refer to clinical intervention in an attempt to prevent, cure, delay, reduce the severity of, or ameliorate one or more symptoms of the disease or disorder or recurring disease or disorder, or in order to prolong the survival of a patient beyond that expected in the absence of such treatment.

The term "colorectal cancer" has been described in detail in the context of the prognostic methods of the invention and is used with the same meaning in the context of the personalized methods according to the invention.

In a first step, the second personalized therapeutic method according to the invention comprises the determination of the expression level of the NPR3/C5orf23, CDKN2B and FLT1 genes in a sample from said patient.

The terms "colorectal cancer", "patient", "the NPR3/C5orf23 gene", "CDKN2B gene", "FLT1 gene", "expression levels", "sample", and "therapy" have been described in detail above and are equally applied to the methods according to the present method.

In yet another preferred method, the second personalized therapeutic method according to the invention further comprises, in addition to the determination of the expression levels of the NPR3/C5orf23, CDKN2B and FLT1 genes, the determination of the expression levels of one or more genes selected from the group consisting of FRMD6, IGFBP3, ESM1, FGF1, GEM, MEX3B, WNT2, NGF, MSC, SETBP1, FLJ10357, DACT, MURC and Col10A1 wherein increased expression levels of one or more of said genes with respect to a reference value for one or more of said genes is indicative that the patient is candidate for receiving adjuvant therapy after surgical treatment wherein decreased expression levels of said genes with respect to a reference value for one or more of said genes is indicative that the patient is not a candidate for receiving radiotherapy or chemotherapy after surgical treatment.

In a preferred embodiment, the second personalized therapeutic method according to the invention comprises the determination of the expression levels of genes CDKN2B, NPR3/C5orf23, FLT1, FRMD6, IGFBP3 and ESM1 and the determination is carried out in a sample from a patient suffering from stage II colorectal cancer.

In another preferred embodiment, the second personalized therapeutic method according to the invention comprises the determination levels of the CDKN2B, NPR3/C5orf23, FLT1, FGF1, GEM, and MEX3B genes and said determination is carried out in a patient suffering from stage III colorectal cancer.

The terms referring to each of these genes have been described in detail above and are equally applied to the methods according to the present method.

In yet another embodiment, the second personalized therapeutic method according to the invention comprises the determination of the expression levels of the of the genes shown in Table 1 having a FC value higher than 2 in the CAFs enriched vs. EPCAM+ column wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment.

Thus, in another embodiment, the second personalized therapeutic method according to the invention further comprise determination of the expression levels of the ANGPTL2, ANGPTL4, APBB2, BMPR2, BPGM, C13orf33, C5orf13, NPR3/C5orf23, CACHD1, CALD1, CDH6, CDKN2B, CILP, CNTN1, COL10A1, COL12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A and ZEB1 genes and to the genes which hybridize specifically with the probes having the sequences SEQ ID NO:1 to 13 wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment.

The term "specifically hybridizing", as used herein, refers to conditions which allow the hybridization of two polynucleotide sequences under high stringent conditions or moderately stringent conditions. The expressions "high stringent conditions" and "moderately stringent conditions" are defined below in respect to the kit of the invention and are equally applicable in the context of the present method.

The expression levels of the different genes used in the second personalized therapeutic method of the invention can be determined by determining the levels of the mRNA encoded by said genes or by determining the levels of the polypeptide encoded by said genes.

In a second step, the personalized therapeutic method according to the invention comprises the identification of those patients showing increased expression levels of said genes with respect to a reference value for said genes as patients who are likely to benefit from therapy after surgical treatment or of those patients showing decreased expression levels of said genes with respect to a reference value for said genes as patients who are unlikely to benefit from therapy after surgical treatment.

The term "benefit" relates to improving the disease state of the patient. Beneficial or desired clinical results include, but not limiting, release of symptoms, reduction of the length of the disease, stabilized pathological state (specifically not deteriorated), retard in the disease's progression, improve of the pathological state, prolongation of survival compared to the expected survival if the treatment is not applied, and remission (both partial and total), both detectable and not detectable.

In a particular embodiment, the therapy after surgical treatment for which the patient is or is not candidate is a therapy selected from the group consisting of chemotherapy, radiotherapy and/or a therapy comprising a TGF-beta inhibitor.

The terms "surgery" or "surgical treatment", "chemotherapy", "chemotherapeutic drug", "radiotherapy", and "therapy comprising a TGF-beta inhibitor" have been described in detail above and are equally applied to the methods according to the present method.

### Personalized medicine based on the expression levels of TGF-beta2 and TGF-beta3 (not part of the invention)

Further disclosed is a method (hereinafter "third personalized therapeutic method") for selecting a suitable treatment of colorectal cancer in a patient comprising the determination in a sample from said patient of the expression levels of the TGF-beta2 and/or TGF-beta3 genes wherein increased expression levels of said gene or genes with respect to a reference value for said genes is indicative that the patient is candidate for receiving adjuvant radiotherapy or chemotherapy after surgical treatment or wherein decreased expression levels of said gene or genes with respect to a reference value for each gene is indicative that the patient is not candidate for receiving adjuvant radiotherapy or chemotherapy after surgical treatment.

The terms "selecting a treatment", "colorectal cancer", "sample, "patient", "TGF-beta2", "TGF-beta3", "increased expression levels", "adjuvant therapy", "radiotherapy", "chemotherapy" have been described above in the context of the prognostic methods and personalized medicine according to the invention and are equally applied to the present method.

In a preferred disclosure, the third personalized therapeutic method is carried out in order to determine whether a patient is candidate for receiving chemotherapy or radiotherapy. In yet another disclosure, the chemotherapy is based on the use of TGF-beta inhibitors as described above.

In a preferred disclosure, the third personalized therapeutic method further comprises determining the tumor stage in the patient wherein high tumor stage is indicative that the patient is candidate for receiving adjuvant radiotherapy or chemotherapy after surgical treatment or wherein a low tumor stage is indicative that the patient shows low risk of recurrence.

In yet another disclosure, the third personalized therapeutic method is carried out in patients wherein the colorectal cancer is a stage II or stage III colorectal tumor.

In yet another disclosure, the expression levels of the TGF-beta2 and/or TGF-beta3 genes are determined by determining the mRNA levels for said genes. In a yet more preferred disclosure, the determination of the levels of the mRNA is carried out by RT-PCR. In another disclosure, the determination of the expression levels of the TGF-beta2 and/or TGF-beta3 genes is carried out by determining the levels of the proteins encoded by said genes.

In another disclosure, the sample wherein the determination of the expression levels of the genes is carried out is selected from the group consisting of a tumor biopsy or a biofluid. In a still more preferred disclosure, wherein the sample is a biofluid, then the biofluid is selected from the group consisting of blood, plasma and serum.

In yet another disclosure, the chemotherapy is based on a TGF-beta inhibitor. The term "TGF-beta inhibitor" has been defined above.

### Personalized therapies of the invention

The prognostic method and the personalized therapeutic methods defined above also allow providing personalized therapies to patients suffering colorectal cancer. In particular, patients which are considered as having a high risk of relapse will most likely benefit from an additional therapy after surgery. Conversely, patients showing low risk of relapse may do without additional therapeutic treatment following surgery.

Thus, in another aspect, the invention relates to a chemotherapeutic agent for use in the treatment of colorectal cancer in a patient after surgical treatment of the cancer, wherein the patient has been selected by a method according to the first aspect of the invention and wherein the chemotherapeutic agent comprises a TGF-beta inhibitor.

The terms "therapy", "treatment", "colorectal cancer", "patient", "radiotherapy", "surgery" or "surgical treatment", "chemotherapy", "chemotherapeutic drug", "radiotherapy", and "therapy comprising a TGF-beta inhibitor" have been described in detail above and are equally applied to the personalized therapies of the invention.

### Kits of the invention

In another aspect, the invention relates to a kit which is useful for the determination of the expression levels of the genes forming the F-TBRS signature, or the minisignatures derived from the F-TBRS. Thus, in an aspect, the kit of the invention comprises reagents adequate for the determination of the expression level of the NPR3/C5orf23, the CDKN2B and the FLT1 genes and, optionally, reagents for the determination of the expression levels of one or more housekeeping genes, wherein the reagents adequate for determining the expression levels of the NPR3/C5orf23, CDKN2B and FLT1 genes comprise at least 10% of the probes present in the kit.

In an embodiment, the kit of the invention comprises reagents adequate for the determination of the expression levels of the NPR3/C5orf23, the CDKN2B, the FLT1 genes and one or more additional genes selected from the group consisting of FRMD6, IGFBP3, ESM1, FGF1, GEM, MEX3B, WNT2, NGF, MSC, SETBP1, FLJ10357, DACT, MURC and Col10A1 genes.

In another embodiment, the kit according to the invention comprises reagents adequate for the determination of the expression levels of the CDKN2B, NPR3/C5orf23, FLT1, FRMD6, IGFBP3 and ESM1 genes.

In another embodiment, the kit according to the invention comprises reagents adequate for the determination of the expression levels of the CDKN2B, NPR3/C5orf23, FLT1, FGF1, GEM, and MEX3B genes.

In another embodiment, the kit according to the present invention further comprises reagents adequate for the determination of the expression levels of one or more of the genes shown in Table 1 which are differentially expressed between the cell population enriched in cancer associated fibroblasts (enriched CAFs) and EPCAM+ and having at least a 2-fold increase in said first cell population.

In another embodiment, the kit comprises reagents adequate for the determination of the expression levels of the ANGPTL2, ANGPTL4, APBB2, BMPR2, BPGM, C13orf33, C5orf13, NPR3/C5orf23, CACHD1, CALD1, CDH6, CDKN2B, CILP, CNTN1, COL10A1, COL12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A and ZEB1 genes and to the genes which hybridize specifically with the probes having the sequences SEQ ID NO:1 to 13.

In a preferred embodiment, the reagents adequate for the determination of the expression levels of one or more genes comprise at least 10%, at least 20%, at least 30%, at least 40%, at least 50%, at least 60%, at least 70%, at least 80%, at least 90% or at least 100% of the total amount of reagents adequate for the determination of the expression levels of genes forming the kit. Thus, in the particular case of kits comprising reagents for the determination of the expression levels of the NPR3/C50RF23, the CDKN2B and the FLT1 genes, the reagents specific for said gene (e.g. probes which are capable of hybridizing under stringent conditions to the NPR3/C50RF23 gene, the CDKN2B and the FLT1 genes) comprise at least 10%, at least 20%, at least 30%, at least 40%, at least 50%, at least 60%, at least 70%, at least 80%, at least 90% or at least 100% of the probes present in the kit.

In further embodiments, the reagents adequate for the determination of the expression levels of one or more genes comprise at least 55% at least 60%, at least 65%, at least 70%, at least 75%, at least 80%, at least 90%, at least 95%, at least 96%, at least 97%, at least 98% or at least 99% of the total amount of reagents forming the kit.

In the context of the present invention, "kit" is understood as a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

In a particular embodiment of the kit of the invention, the reagents of the kit are nucleic acids which are capable of specifically detecting the mRNA level of the genes mentioned above and/or the level of proteins encoded by one or more of the genes mentioned above. Nucleic acids capable of specifically hybridizing with the genes mentioned above can be one or more pairs of primer oligonucleotides for the specific amplification of fragments of the mRNAs (or of their corresponding cDNAs) of said genes.

In a preferred embodiment, the first component of the kit of the invention comprises a probe which can specifically hybridize to the genes mentioned above.

The term "specifically hybridizing", as used herein, refers to conditions which allow hybridizing of two polynucleotide under high stringent conditions or moderately stringent conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C., with washes at 42°C in 0.2xSSC (sodium chloride/sodium citrate) and 50% formamide, followed by a high-stringency wash consisting of 0.1xSSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C. in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, etc. as necessary to accommodate factors such as probe length and the like.

In the event that the expression levels of several of the genes identified in the present invention are to be simultaneously determined, it is useful to include probes for all the genes the expression of which is to be determined in a microarray hybridization.

The microarrays comprise a plurality of nucleic acids that are spatially distributed and stably associated to a support (for example, a biochip). The nucleic acids have a sequence complementary to particular subsequences of genes the expression of which is to be detected, therefore are capable of hybridizing with said nucleic acids. In the methods of the invention, a microarray comprising an array of nucleic acids is put into contact with a preparation of nucleic acids isolated from the patient object of the study. The incubation of the microarray with the preparation of nucleic acids is carried out in conditions suitable for the hybridization. Subsequently, after the elimination of the nucleic acids which have not been retained in the support, the hybridization pattern is detected, which provides information on the genetic profile of the sample analyzed. Although the microarrays are capable of providing both qualitative and quantitative information of the nucleic acids present in a sample, the invention requires the use of arrays and methodologies capable of providing quantitative information.

The invention contemplates a variety of arrays with regard to the type of probes and with regard to the type of support used. The probes included in the arrays that are capable of hybridizing with the nucleic acids can be nucleic acids or analogs thereof which maintain the hybridization capacity such as for example, nucleic acids in which the phosphodiester bond has been substituted with a phosphorothioate, methylimine, methylphosphonate, phosphoramidate, guanidine bond and the like, nucleic acids in which the ribose of the nucleotides is substituted with another hexose, peptide nucleic acids (PNA). The length of the probes can of 5 to 50 nucleotides and, preferably, of 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-stranded or doublestranded nucleic acids. The array can contain all the specific probes of a certain mRNA of a certain length or can contain probes selected from different regions of an mRNA. Each probe is assayed in parallel with a probe with a changed base, preferably in a central position of the probe. The array is put into contact with a sample containing nucleic acids with sequences complementary to the probes of the array and the signal of hybridization with each of the probes and with the corresponding hybridization controls is determined. Those probes in which a higher difference is observed between the signal of hybridization with the probe and its hybridization control are selected. The optimization process can include a second round of optimization in which the hybridization array is hybridized with a sample that does not contain sequences complementary to the probes of the array. After the second round of selection, those probes having signals of hybridization lower than a threshold level will be selected. Thus, probes which pass both controls, i.e., which show a minimum level of unspecific hybridization and a maximum level of specific hybridization with the target nucleic acid are selected.

The selection of the specific probes for the different target genes is carried out such that they bind specifically to the target nucleic acid with a minimum hybridization to non-related genes. However, there are probes of 20 nucleotides which are not unique for a certain mRNA. Therefore, probes directed to said sequences will show a cross-hybridization with identical sequences that appear in mRNA of non-related genes. In addition, there are probes that do not specifically hybridize with the target genes in the conditions used (because of secondary structures or of interactions with the substrate of the array). This type of probe must not be included in the array. Therefore, the person skilled in the art will observe that the probes that are going to be incorporated in a certain array must be optimized before their incorporation to the array. The optimization of the probes is generally carried out by generating an array containing a plurality of probes directed to the different regions of a certain target polynucleotide. This array is put into contact firstly with a sample containing the target nucleic acid in an isolated form and, secondly, with a complex mixture of nucleic acids. Probes which show a highly specific hybridization with the target nucleic acid but low or no hybridization with the complex sample are thus selected for their incorporation to the arrays of the invention. Additionally, it is possible to include in the array hybridization controls for each of the probes that is going to be studied. In a preferred embodiment, the hybridization controls contain an altered position in the central region of the probe. In the event that high levels of hybridization are observed between the studied probe and its hybridization control, the probe is not included in the array.

The microarrays of the invention contain not only specific probes for the polynucleotides indicating a determined pathophysiological situation, but also containing a series of control probes, which can be of three types: normalization controls, expression level controls and hybridization controls.

Normalization controls are oligonucleotides that are perfectly complementary to labeled reference sequences which are added to the preparation of nucleic acids to be analyzed. The signals derived from the normalization controls after the hybridization provide an indication of the variations in the hybridization conditions, intensity of the marker, efficiency of the detection and another series of factors that can result in a variation of the signal of hybridization between different microarrays. The signals detected from the rest of probes of the array are preferably divided by the signal emitted by the control probes, thus normalizing the measurements. Virtually any probe can be used as normalization control. However, it is known that the efficiency of the hybridization varies according to the composition of nucleotides and the length of the probe. Therefore, preferred normalization probes are those which represent the mean length of the probes present in the array, although they can be selected such that they include a range of lengths that reflect the rest of probes present in the array. The normalization probes can be designed such that they reflect the mean composition of nucleotides of the rest of probes present in the array. A limited number of normalization probes is preferably selected such that they hybridize suitably, i.e., they do not have a secondary structure and do not show sequence similarity with any of the probes of the array is used. The normalization probes can be located in any position in the array or in multiple positions in the array to efficiently control variations in hybridization efficiency related to the structure of the array. The normalization controls are preferably located in the corners of the array and/or in the center thereof.

The expression controls levels are probes which hybridize specifically with genes which are expressed constitutively in the sample which is analyzed. The expression level controls are designed to control the physiological state and the metabolic activity of the cell. The examination of the covariance of the expression level control with the expression level of the target nucleic acid indicates if the variations in the expression levels are due to changes in the expression levels or are due to changes in the overall transcriptional rate in the cell or in its general metabolic activity. Thus, in the case of cells which have deficiencies in a certain metabolite essential for cell viability, the observation of a decrease both in the expression levels of the target gene as in the expression levels of the control is expected. On the other hand, if an increase in the expression of the expression of the target gene and of the control gene is observed, it probably due to an increase of the metabolic activity of the cell and not to a differential increase in the expression of the target gene. Probes suitable for use as expression controls correspond to genes expressed constitutively, such as genes encoding proteins which exert essential cell functions such as β-2-microglobulin, ubiquitin, ribosomal protein 18S, cyclophilin A, transferrin receptor, actin, GAPDH, tyrosine 3-monooxygenase/tryptophan 5-monooxygenase activation protein (YWHAZ) and beta-actin.

Hybridization controls can be included both for the probes directed to target genes and for the probes directed to the expression level or to the normalization controls. Error controls are probes of oligonucleotides identical to the probes directed to target genes but which contain mutations in one or several nucleotides, i.e., which contain nucleotides in certain positions which do not hybridize with the corresponding nucleotide in the target gene. The hybridization controls are selected such that, applying the suitable hybridization conditions, the target gene should hybridize with the specific probe but not with the hybridization control or with a reduced efficiency. The hybridization controls preferably contain one or several modified positions in the center of the probe. The hybridization controls therefore provide an indication of the degree of unspecific hybridization or of cross-hybridization to a nucleic acid in the sample to a probe different from that containing the exactly complementary sequence.

The arrays of the invention can also contain amplification and sample preparation controls which are probes complementary to subsequences of selected control genes because they normally do not appear in the biological sample object of the study, such as probes for bacterial genes. The RNA sample is supplemented with a known amount of a nucleic acid which hybridizes with the selected control probe. The determination of the hybridization to said probe indicates the degree of recovery of the nucleic acids during their preparation as well as an estimation of the alteration caused in the nucleic acids during the processing of the sample.

Once a set of probes showing the suitable specificity and a set of control probes are provided, the latter are arranged in the array in a known position such that, after the steps of hybridization and of detection, it is possible to establish a correlation between a positive signal of hybridization and the particular gene from the coordinates of the array in which the positive signal of hybridization is detected.

The microarrays can be high density arrays with thousands of oligonucleotides by means of photolithographic in situ synthesis methods (Fodor et al., 1991, Science, 767-773). This type of probe is usually redundant, i.e., they include several probes for each mRNA which is to be detected. In a preferred embodiment, the arrays are low density arrays or LDA containing less than 10000 probes per square centimeter. In said low density arrays, the different probes are manually applied with the aid of a pipette in different locations of a solid support (for example, a crystal surface, a membrane). The supports used to fix the probes can be obtained from a large variety of materials, including plastic, ceramics, metals, gels, membranes, crystals and the like. The microarrays can be obtained using any methodology known for the person skilled in the art.

After the hybridization, in the cases in which the non-hybridized nucleic acid is capable of emitting a signal in step of detection, a step of washing is necessary to eliminate said non-hybridized nucleic acid. The step of washing is carried out using methods and solutions known by the person skilled in the art.

In the event that the labeling in the nucleic acid is not directly detectable, it is possible to connect the microarray comprising the target nucleic acids bound to the array with the other components of the system necessary to cause the reaction giving rise to a detectable signal. For example, if the target nucleic acids are labeled with biotin, the array is put into contact with conjugated streptavidin with a fluorescent reagent in suitable conditions so that the binding between biotin and streptavidin occurs. After the incubation of the microarray with the system generating the detectable signal, it is necessary to carry out a step of washing to eliminate all the molecules which have bound non-specifically to the array. The washing conditions will be determined by the person skilled in the art using suitable conditions according to the system generating the detectable signal and which are well known for the person skilled in the art.

The resulting hybridization pattern can be viewed or detected in several different ways, said detection being determined by the type of system used in the microarray. Thus, the detection of the hybridization pattern can be carried out by means of scintillation counting, autoradiography, determination of a fluorescent signal, calorimetric determinations, detection of a light signal and the like.

After the hybridization and the possible subsequent washing and treatment processes, the hybridization pattern is detected and quantified, for which the signal corresponding to each point of hybridization in the array is compared to a reference value corresponding to the signal emitted by a known number of terminally labeled nucleic acids in order to thus obtain an absolute value of the number of copies of each nucleic acid which is hybridized in a certain point of the microarray.

In the event that the expression levels of the genes according to the present invention is determined by measuring the levels of the polypeptide or polypeptides encoded by said gene or genes, the kits according to the present invention comprise reagents which are capable of specifically binding to said polypeptide or polypeptides. Thus, in one embodiment, the invention relates to a kit comprising antibodies specific for the polypeptides encoded by the NPR3/C5orf23, the CDKN2B and the FLT1 genes.

In another embodiment, the kit of the invention comprises antibodies specific for the NPR3/C5orf23, the CDKN2B, the FLT1 genes and for one or more additional polypeptides selected from the group consisting of the polypeptides encoded by the FRMD6, IGFBP3, ESM1, FGF1, GEM, MEX3B, WNT2, NGF, MSC, SETBP1, FLJ10357, DACT, MURC and Col10A1 genes.

In another embodiment, the kit according to the invention comprises antibodies specific for the CDKN2B, NPR3/C5orf23, FLT1, FRMD6, IGFBP3 and ESM1 genes.

In another embodiment, the kit according to the invention comprises antibodies specific for the CDKN2B, NPR3/C5orf23, FLT1, FGF1, GEM, and MEX3B genes.

In another embodiment, the kit according to the present invention further comprises antibodies specific for the polypeptides encoded by the genes shown in Table 1 which are differentially expressed between the cell population enriched in cancer associated fibroblasts (enriched CAFs) and EPCAM+ and having at least a 2-fold increase in said first cell population.

In another embodiment, the kit according to the present invention comprises antibodies specific for the polypeptides encoded by the to the ANGPTL2, ANGPTL4, APBB2, BMPR2, BPGM, C13orf33, C5orf13, NPR3/C5orf23, CACHD1, CALD1, CDH6, CDKN2B, CILP, CNTN1, COL10A1, COL12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A and ZEB1 genes and to the genes which hybridize specifically with the probes having the sequences SEQ ID NO:1 to 13.

For this purpose, the arrays of antibodies such as those described by De Wildt et al. (2000) Nat. Biotechnol. 18:989-994; Lueking et al. (1999) Anal. Biochem. 270:103-111; Ge et al. (2000) Nucleic Acids Res. 28, e3, I-VII; MacBeath and Schreiber (2000) Science 289:1760-1763; WO 01/40803 and WO 99/51773A1 are useful. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like. The techniques for preparing said antibodies are very well known for the person skilled in the art and include the methods described by Ausubel et al. (Current Protocols in Molecular Biology, eds. Ausubel et al, John Wiley & Sons (1992)).

The antibodies of the array can be applied at high speed, for example, using commercially available robotic systems (for example, those produced by Genetic Microsystems or Biorobotics). The substrate of the array can be nitrocellulose, plastic, crystal or can be of a porous material as for example, acrylamide, agarose or another polymer. In another embodiment, it is possible to use cells producing the specific antibodies for detecting the proteins of the invention by means of their culture in array filters. After the induction of the expression of the antibodies, the latter are immobilized in the filter in the position of the array where the producing cell was located. An array of antibodies can be put into contact with a labeled target and the binding level of the target to the immobilized antibodies can be determined. If the target is not labeled, a sandwich type assay can be used in which a second labeled antibody specific for the polypeptide which binds to the polypeptide which is immobilized in the support is used. The quantification of the amount of polypeptide present in the sample in each point of the array can be stored in a database as an expression profile. The array of antibodies can be produced in duplicate and can be used to compare the binding profiles of two different samples.

In another aspect, the invention relates to the use of a kit of the invention for predicting the outcome of a patient suffering colorectal cancer or for selecting a patient which is likely to benefit from adjuvant therapy after surgical resection of colorectal cancer. In a preferred embodiment, the use of the kits according to the invention is carried out in patients suffering stage II or stage III CRC.

The invention is detailed below by means of the following examples which are merely illustrative and by no means limiting for the scope of the invention.

### EXAMPLES

### Materials and methods

### Clinical material

Human tissue samples were obtained from the Pathology Department of Hospital del Mar with the approval of the Bank Tumor Committee according to Spanish Ethical regulations. The study followed the guidelines of the Declaration of Helsinki and patient's identity of pathological specimens remained anonymous in the context of this study. Human colon normal fibroblasts (CCD-18Co) were obtained from ATCC and cultured for less than 10 passages prior to experiments.

### Classification of tumors samples according to p-Smad3 staining

Adenomas (n = 25) and CRC (n = 30) samples routinely collected at Hospital del Mar were stained with anti-p-SMAD3 antibody. Qualitative categorization of the samples according overall p-SMAD3 intensity or staining in tumor-associated stroma and epithelial cancer cells was performed by an expert pathologist (M.I). Average staining levels were summarized into three categories; high, medium and low. For tumor-associated stroma, all cell types were taken into consideration.

### Tumor disaggregation and staining

Freshly obtained tumors from CRC patients (n=8) treated at Hospital del Mar (Barcelona, Spain) were minced with sterile razor blade and incubated with rotation for 15-20 minutes at 37°C in 50% DMEM / 50% F12 (both from Gibco), containing 100X penicillin/streptomycin (Gibco); 0.1% Hyaluronidase and 0.1% Collagenase 1A (both from Sigma). Pieces were then homogenized by pipetting and passed through consecutive 18G and 21G needles. Enzymatic reaction was stopped by adding 10% FBS and single cells were collected by sequential filtering through cell strainers of 100 µm → 70 µm → 40 µm (BD Falcon). Cells were centrifuged, resuspended in 5 ml Ammonium Chloride (0.15M; Sigma Aldrich) and incubated 5 minutes at room temperature to lyse erythrocytes. After two washes with HBSS (Lonza) cells were incubated for 5 minutes in 1 ml blocking solution: staining buffer (SB) (HBSS + 5% FBS); 1% BSA; 5% mouse serum. Cells were then stained in SB with anti-hEpcam/TROP1-APC conjugated antibody (30 min; 1/50; R&D Systems) and anti-CD45-PE conjugated antibody (20 min; 1/10; Miltenyi Biotec). Dead cells were labeled with Propidium Iodide.

Fluorescence Activated Cell Sorting (FACS) was used to separate the cells. RNA was extracted using the RNeasy kit (Qiagen). Labeling and hybridization of samples to HG-U133A 2.0 gene expression chips (Affymetrix) were performed by IRB Transcriptomic Core Facility using standard methodology. Data analysis was performed using Partek software.

### Generation of F-TBRS gene expression signature

CCD-18Co Fibroblasts were seeded at 60% confluence and treated with TGFB1 (Peprotech; 5 ng/ml) for 8 hours. Gene expression profiles were measured using HG-U133 plus 2.0 Affimetrix arrays and normalized via RMA. A first signature was generated that contained genes up-regulated at least 2 fold in fibroblasts treated with TGF-beta in duplicate experiments (p<0.05). This list was further refined by filtering through the expression profiles of cell populations purified from CRC patients: [CD45(+), Epcam(-): Leukocytic fraction], [CD45(-) Epcam(+); epithelial fraction] and [CD45(-) Epcam(-); CAF enriched fraction]. The F-TBRS corresponds to genes upregulated (>2 fold, p<0.05) in the CAF enriched fraction compared to the other two populations. All p-values for fold change enrichment were obtained via moderated t-tests.

### Datasets

Datasets corresponding to human colon adenomas and carcinomas have been previously described (Sabates-Bellver et al., 2007; Mol. Cancer Res., 5, 1263-1275; van der Flier et al., 2007; Gastroenterology, 132, 628-632). To correlate F-TBRS expression with clinical disease progression, we pooled two sets of Affymetrix transcriptomic profiles (GSE17537 and GSE14333), available at Gene Expression Omnibus, www.ncbi.nlm.nih.gov/geo, corresponding to primary CRCs for which clinical follow-up was available. GSE1753729 is composed of 55 colon cancer patients treated at Vanderbilt University Medical Center (Vanderbilt, USA). GSE1433330 contains a pool of 290 CRC patients treated at two different hospitals; Peter MacCallum Cancer Center (Australia) and H. Lee Moffitt Cancer Center (USA). Available annotated clinical data for GSE17537 and GSE14333 datasets included AJCC staging, age, gender and disease free survival intervals. The representation of tumor samples at different AJCC stages in these cohorts follows the natural distribution of CRC patients receiving standard treatment in the aforementioned hospitals. In order to remove systematic biases between datasets, expression levels for all genes were transformed to z-scores prior to pooling.

For in silico validation studies two additional cohorts were used, datasets GSE33113 and GSE37892. GSE33113 contained a set of 90 AJCC stage II CRC patient material collected in the Academic Medical Center (AMC) in Amsterdam, The Netherlands. Extensive medical records were kept from these patients and long-term clinical follow-up was available for the large majority. The cohort in GSE37892 contained a series of 130 colon cancer samples in which there were both stage II and stage III CRC patients.

### Association of F-TBRS and clinical outcome

For association of F-TBRS with disease free survival we did not take into consideration Stage IV CRCs as relapse in these cases is often associated with incomplete surgical resection of the tumor at the metastatic site. We used gene set enrichment analysis (GSEA) to assess the degree of association between our signature and associated variables. GSEA was based on ranking genes according to their hazard ratios (estimated via Cox model) for disease relapse and according to fold change for the remaining variables. The output of GSEA is an enrichment score (ES), a normalized enrichment score (NES) which accounts for the size of the gene set being tested, a P-value and an estimated False Discovery rate (FDR). ES, NES and FDR were obtained as proposed in Subramanian et al (Proc. Natl. Acad. Sci. USA., 2005, 102:15545-15550). P-values were computed using 10,000 permutations for each signature and adjusted them with the Benjamini-Yekutieli method (Behav. Brain Res., 2001, 125, 279-284). To assess the signature's predictive power on recurrence we computed the mean signature expression and tested its significance with a univariate Cox proportional hazards model likelihood ratio test. The mean signature expression was computed by obtaining z-scores for each gene and averaging z-scores across all genes in the signature. Patients were divided into three groups according to their mean F-TBRS expression: F-TBRS low (expression < M - SD), F-TBRS medium (expression > M - SD and < M) and F-TBRS high (expression > M), where M is the average across all patients and SD the standard deviation. We obtained Kaplan-Meier survival curves for patients with low, medium and high average signature scores. The multivariate Cox model also included age, gender and AJCC stage. Non-significant variables were dropped from the model in a stepwise fashion until all variables were statistically significant. Statistical significance was defined at the 0.05 level. P-values in all Cox models were based on likelihood ratio tests.

A SCAD-based logistic regression model was fitted (Fan & Li, 1999, Journal of the American Statistical Association, 1999, 96, 1348-1360) to predict recurrence events based on patient age, gender, staging and gene expression, and selected the variables with non-zero coefficient estimates. The SCAD penalization parameter was set via 10-fold cross-validation, as implemented in the R package ncvreg. We performed this analysis for stage II, III and also for all patients, which provided several short and highly predictive gene signatures.

In order to further assess the association of each gene signature and disease-free survival, the average signature expression was computed (i.e. across all genes in the signature) and fit multivariate Cox proportional-hazards models that included staging as an adjustment variable. To visualize the results we stratified the patients according to their average signature expression and obtained Kaplan-Meier plots, and estimated the effect on the hazard ratio as a smooth function using quartic penalized splines (Eilers et al, 1996, Statistical Science, 11, 89-121) as implemented in the R package pspline.

### Immunohistochemistry

Immunohistochemistry was performed on paraffin sections using primary antibodies raised against phospho-SMAD3 (Rockland). Briefly, sections were autoclaved 10 minutes in citrate buffer pH 6 previous to incubation with anti phospho-smad 3 (1/100). Secondary biotinylated antibodies (Vector Laboratories Inc) were used at a 1:200 dilution and detected using the Vectastain ABC kit (Vector Laboratories Inc), as recommended by the supplier.

### EXAMPLE 1

### TGF-beta signaling during CRC progression

TGF-beta signaling during CRC progression was explored. Gene expression profiling of colon tumor samples confirmed elevated levels of *TGFB1*, *TGFB2* and *TGFB3* mRNAs in a subset of CRCs whereas all adenomas displayed low levels of the three TGF-beta isoforms (Fig. 1). Characteristic features of the adenoma-CRC transition include increased desmoplastic reaction, inflammation and neovascularization, all of which involve several non-cancerous cell types that reside within the tumor stroma.

To study the cell-type specific expression of TGF-beta isoforms, specific tumor cell populations purified from primary CRCs were profiled (n=8 patients). Analysis of marker gene expression indicated that EPCAM+ cell population was largely enriched in epithelial cancer cells, the EpCAM-/CD45+ cell population was enriched in leukocytes and the EPCAM-/CD45- cell population in cancer-associated fibroblasts (CAFs) (data not shown). TGFB1 expression was high in non-epithelial cells (both CD45+ and CD45-) whereas elevated TGFB2 and TGFB3 mRNA levels were only present in the CAF-enriched cell population (Fig. 2).

To identify cell populations targeted by TGF-beta in adenomas and CRCs, phosphorylation of SMAD3 (p-SMAD3) was used as a marker of TGF-beta pathway activation. Immunohistochemistry analysis on clinical material revealed prominent nuclear p-SMAD3 accumulation in epithelial cancer cells in 40% of the adenomas but only in 7% of the CRCs (Fig. 3). This finding may reflect the frequent acquisition of inactivating mutations in TGF-beta signaling pathway components during CRC progression. Additionally, epithelial p-SMAD3+ CRC cells may have impaired TGF-beta transcriptional response due to genetic alterations in SMAD4 (Liu et al., 1997, Genes dev., 11: 3157-3167 and Alarcon, C., 2009, Cell, 139: 757-769). Concurrent with the loss of epithelial TGF-beta signaling in CRCs, tumor stromal cells displayed high levels of p-SMAD3 (Fig. 3). While the stroma of most adenomas contained few p-SMAD3 highly positive cells and stained weakly overall, a large proportion of CRCs (63%) were characterized by an abundance of stromal cells with strong nuclear p-SMAD3 staining. Altogether, these observations suggest that a subset of colon tumors undergoes an increase in the expression of TGF-beta at the adenoma-carcinoma transition, and tumor stroma components are the main contributors to this increase. Elevated levels of TGF-beta in CRCs preferentially target tumor-associated stromal cells rather than the cancer cells.

### EXAMPLE 2

### Identification of a fibroblast-specific TGF-beta response Signature (F-TBRS) capable of predicting outcome of CRC.

As shown in example 1, p-SMAD3 accumulated in the nucleus of different tumor stromal-associated cells including lymphocytes and endothelial cells. Yet, the most common stromal p-SMAD3+ cell-type in CRCs displayed a thin and elongated shape with numerous membrane extensions characteristic of fibroblasts. Cancer-Associated Fibroblasts (CAFs) are the most abundant population of the reactive cancer stroma and they participate in tumor growth, invasion and metastasis in several cancer types. To investigate the role of TGF-beta-stimulated fibroblasts in CRC, the set of genes regulated by TGF-beta in these cells was first identified. Normal colon fibroblasts (CCD-18Co) were cultured in the presence or absence of TGF-beta and global gene expression profiles were assessed using microarrays. TGF-beta signalling up-regulated the expression levels of 280 genes in these cells (391 probes; >2 fold, p<0.05). The cell-type specificity of this gene set was assessed using the purified tumor cell populations described in example 1. Out of the 391 probes induced by TGF-beta in CCD-18Co fibroblasts, 127 genes (detected by 175 probes) were differentially upregulated in the CAF-enriched cell population (Fig. 4; Table 1). These 175 probes (127 genes) were termed Fibroblast-specific TGF-beta Response Signature (F-TBRS). Remarkably, F-TBRS was highly expressed in CRCs compared with adenomas (data not shown). This observation is consistent with the increased levels of *TGFB1*, 2 and 3 and increased abundance of p-SMAD3+ CAFs during CRC progression (Fig.1).

**Table 1: List of genes differentially regulated in TGF-beta stimulated colon fibroblasts wherein "CCD+ vs. CCD- fold change" refers to the fold change in gene expression level between TGF-beta stimulated CAF and normal colon fibroblasts and wherein "CD45-/Epcam- vs. CD45+ and Epcam+ Fold-Change" refers to the fold change in the gene expression between TGF-beta stimulated CAF and the average gene expression in Epcam+ (epithelial) cells and Epcam-Cd45+ cells (Leukocytes). NA: Not annotated.**

| **affyid** | **Gene symbol** | **CCD+ vs. CCD-Fold-Change** | **CD45-/Epcam-vs. CD45+ and Epcam+ Fold-Change** |
|---|---|---|---|
| 219514_at | ANGPTL2 | 1,8 | 13,5 |
| 221009_s_at | ANGPTL4 | 2,9 | 2,4 |
| 223333_s_at | ANGPTL4 | 3,4 | 1,9 |
| 212985_at | APBB2 | 2,4 | 11,4 |
| 213419_at | APBB2 | 2,6 | 6,1 |
| 40148_at | APBB2 | 2,4 | 5,2 |
| 210214_s_at | BMPR2 | 2,1 | 3,9 |
| 238516_at | BMPR2 | 2,1 | 1,8 |
| 203502_at | BPGM | 2,0 | 2,3 |
| 227058_at | C13orf33 | 2,5 | 22,8 |
| 230424_at | C5orf13 | 2,2 | 3,7 |
| 219054_at | NPR3/C5orf23 | 1,7 | 6,5 |
| 225627_s_at | CACHD1 | 4,2 | 8,7 |
| 205525_at | CALD1 | 2,6 | 19,2 |
| 215199_at | CALD1 | 2,1 | 22,9 |
| 205533_s_at | CDH6 | 2,1 | 8,1 |
| 210602_s_at | CDH6 | 2,0 | 4,5 |
| 236313_at | CDKN2B | 7,9 | 3,3 |
| 206227_at | CILP | 3,1 | 11,2 |
| 227209_at | CNTN1 | 2,8 | 4,2 |
| 205941_s_at | COL10A1 | 1,9 | 6,6 |
| 217428_s_at | COL10A1 | 2,7 | 20,7 |
| 233109_at | COL12A1 | 1,5 | 5,8 |
| 225288_at | COL27A1 | 2,9 | 8,0 |
| 225293_at | COL27A1 | 2,1 | 5,9 |
| 219179_at | DACT1 | 2,8 | 19,6 |
| 214724_at | DIXDC1 | 1,8 | 9,8 |
| 212817_at | DNAJB5 | 2,2 | 2,5 |
| 227166_at | DNAJC18 | 2,1 | 4,7 |
| 219134_at | ELTD1 | 1,9 | 33,1 |
| 227449_at | EPHA4 | 3,0 | 4,8 |
| 228948_at | EPHA4 | 2,7 | 4,0 |
| 229374_at | EPHA4 | 2,0 | 3,7 |
| 208394_x_at | ESM1 | 10,5 | 22,2 |
| 209955_s_at | FAP | 2,0 | 36,4 |
| 1555137_a_at | FGD6 | 1,8 | 2,0 |
| 219901_at | FGD6 | 2,2 | 2,0 |
| 1552721_a_at | FGF1 | 2,8 | 3,6 |
| 205117_at | FGF1 | 2,5 | 19,5 |
| 208240_s_at | FGF1 | 2,2 | 2,0 |
| 204421_s_at | FGF2 | 1,9 | 4,4 |
| 220326_s_at | FLJ10357 | 2,1 | 2,2 |
| 58780_s_at | FLJ10357 | 1,8 | 2,0 |
| 226498_at | FLT1 | 4,6 | 25,9 |
| 214701_s_at | FN1 | 2,5 | 2,9 |
| 214702_at | FN1 | 2,8 | 2,5 |
| 225163_at | FRMD4A | 2,9 | 6,8 |
| 225464_at | FRMD6 | 1,6 | 25,3 |
| 225481_at | FRMD6 | 1,4 | 19,3 |
| 204457_s_at | GAS1 | 3,4 | 23,5 |
| 204472_at | GEM | 1,8 | 3,8 |
| 205100_at | GFPT2 | 1,8 | 14,5 |
| 230369_at | GPR161 | 1,9 | 12,0 |
| 206432_at | HAS2 | 3,6 | 5,4 |
| 230372_at | HAS2 | 3,6 | 14,1 |
| 44783_s_at | HEY1 | 2,3 | 6,2 |
| 230218_at | HIC1 | 2,2 | 5,8 |
| 219985_at | HS3ST3A1 | 1,8 | 8,6 |
| 212143_s_at | IGFBP3 | 1,8 | 30,4 |
| 213910_at | IGFBP7 | 1,7 | 24,3 |
| 206924_at | IL11 | 3,2 | 5,6 |
| 204926_at | INHBA | 2,2 | 2,4 |
| 210511_s_at | INHBA | 2,2 | 11,5 |
| 227140_at | INHBA | 1,7 | 7,1 |
| 205206_at | KAL1 | 2,2 | 14,1 |
| 1561394_s_at | KIAA1755 | 7,0 | 23,2 |
| 204334_at | KLF7 | 2,3 | 2,7 |
| 218651_s_at | LARP6 | 1,8 | 9,5 |
| 218574_s_at | LMCD1 | 7,5 | 7,4 |
| 227317_at | LMCD1 | 2,8 | 7,4 |
| 242767_at | LMCD1 | 5,2 | 6,7 |
| 227155_at | LMO4 | 2,2 | 2,4 |
| 232090_at | LOC100128178 | 3,0 | 23,2 |
| 1558404_at | LOC644242 | 2,3 | 2,8 |
| 227183_at | LOC728264 | 2,5 | 47,7 |
| 231987_at | LOC728264 | 1,8 | 15,2 |
| 220244_at | LOH3CR2A | 8,0 | 4,5 |
| 233487_s_at | LRRC8A | 1,3 | 2,9 |
| 205619_s_at | MEOX1 | 10,2 | 14,6 |
| 223627_at | MEX3B | 3,6 | 1,9 |
| 203417_at | MFAP2 | 1,8 | 21,0 |
| 227488_at | MGC16121 | 2,1 | 2,1 |
| 228235_at | MGC16121 | 5,0 | 3,5 |
| 229784_at | MGC16121 | 2,2 | 2,4 |
| 209928_s_at | MSC | 2,2 | 4,7 |
| 241749_at | MURC | 10,6 | 2,0 |
| 1553275_s_at | NA | 2,0 | 12,7 |
| 1556773_at | NA | 2,1 | 6,7 |
| 225227_at | NA | 1,7 | 1,3 |
| 225328_at | NA | 3,3 | 5,4 |
| 232453_at | NA | 2,2 | 6,1 |
| 232544_at | NA | 1,9 | 16,0 |
| 235629_at | NA | 9,8 | 3,3 |
| 236764_at | NA | 1,7 | 10,1 |
| 237452_at | NA | 1,6 | 3,1 |
| 238617_at | NA | 2,2 | 22,1 |
| 240135_x_at | NA | 2,5 | 5,2 |
| 241272_at | NA | 2,4 | 6,6 |
| 243416_at | NA | 2,7 | 6,0 |
| 214803_at | CDH6 | 1,8 | 71,1 |
| 230254_at | FAM26E | 1,6 | 14,4 |
| 240432_x_at | KLF7 | 1,9 | 2,6 |
| 229669_at | LOC100507263 | 2,9 | 7,6 |
| 237117_at | LOC727930 | 6,0 | 2,3 |
| 226497_s_at | FLT1 | 3,2 | 12,5 |
| 202149_at | NEDD9 | 3,5 | 1,9 |
| 206814_at | NGF | 6,3 | 7,6 |
| 219773_at | NOX4 | 4,3 | 45,8 |
| 236843_at | NOX4 | 3,1 | 10,3 |
| 214066_x_at | NPR2 | 1,7 | 2,5 |
| 204589_at | NUAK1 | 3,0 | 18,0 |
| 204024_at | OSGIN2 | 2,2 | 1,5 |
| 200906_s_at | PALLD | 2,0 | 6,4 |
| 1554640_at | PALM2 | 1,9 | 3,5 |
| 229830_at | PDGFA | 2,6 | 4,1 |
| 222719_s_at | PDGFC | 2,0 | 4,6 |
| 218691_s_at | PDLIM4 | 2,1 | 8,6 |
| 221898_at | PDPN | 1,7 | 27,1 |
| 226658_at | PDPN | 1,9 | 19,7 |
| 229256_at | PGM2L1 | 2,6 | 2,8 |
| 222171_s_at | PKNOX2 | 2,2 | 5,2 |
| 63305_at | PKNOX2 | 1,9 | 8,1 |
| 222450_at | PMEPA1 | 3,5 | 2,8 |
| 201578_at | PODXL | 3,2 | 9,6 |
| 236302_at | PPM1E | 1,6 | 2,4 |
| 206300_s_at | PTHLH | 3,2 | 3,4 |
| 210355_at | PTHLH | 5,5 | 13,2 |
| 211756_at | PTHLH | 2,9 | 11,9 |
| 223467_at | RASD1 | 3,0 | 1,7 |
| 205801_s_at | RASGRP3 | 3,7 | 6,1 |
| 219167_at | RASL12 | 3,3 | 35,9 |
| 204337_at | RGS4 | 2,2 | 27,9 |
| 227657_at | RNF150 | 2,0 | 13,9 |
| 209360_s_at | RUNX1 | 1,7 | 2,6 |
| 230464_at | S1PR5 | 3,0 | 9,1 |
| 226492_at | SEMA6D | 1,9 | 12,4 |
| 1568765_at | SERPINE1 | 6,8 | 9,3 |
| 202627_s_at | SERPINE1 | 2,1 | 16,2 |
| 202628_s_at | SERPINE1 | 2,2 | 10,6 |
| 205933_at | SETBP1 | 3,8 | 4,9 |
| 227478_at | SETBP1 | 4,5 | 6,3 |
| 230493_at | SHISA2 | 1,5 | 40,4 |
| 214719_at | SLC46A3 | 3,4 | 1,7 |
| 219511_s_at | SNCAIP | 3,6 | 9,7 |
| 237833_s_at | SNCAIP | 2,8 | 2,3 |
| 232355_at | SNORD114-3 | 2,6 | 4,5 |
| 227498_at | SOX6 | 2,6 | 15,1 |
| 228214_at | SOX6 | 2,3 | 8,4 |
| 226075_at | SPSB1 | 2,1 | 4,3 |
| 212565_at | STK38L | 3,6 | 2,4 |
| 244070_at | SYNE1 | 2,2 | 9,8 |
| 229991_s_at | SYTL4 | 1,5 | 7,9 |
| 212385_at | TCF4 | 1,9 | 9,6 |
| 213891_s_at | TCF4 | 1,7 | 7,1 |
| 222146_s_at | TCF4 | 1,8 | 9,1 |
| 228837_at | TCF4 | 2,0 | 16,9 |
| 228121_at | TGFB2 | 2,3 | 15,0 |
| 201147_s_at | TIMP3 | 1,8 | 14,7 |
| 201148_s_at | TIMP3 | 1,9 | 14,7 |
| 201149_s_at | TIMP3 | 2,1 | 15,9 |
| 201150_s_at | TIMP3 | 1,8 | 21,1 |
| 229452_at | TMEM88 | 1,8 | 7,7 |
| 216005_at | TNC | 3,7 | 3,8 |
| 218864_at | TNS1 | 1,9 | 8,1 |
| 221747_at | TNS1 | 1,7 | 20,5 |
| 206116_s_at | TPM1 | 1,7 | 5,7 |
| 223392_s_at | TSHZ3 | 2,3 | 7,3 |
| 223393_s_at | TSHZ3 | 2,3 | 11,3 |
| 227233_at | TSPAN2 | 3,4 | 4,6 |
| 227236_at | TSPAN2 | 3,4 | 4,8 |
| 232122_s_at | VEPH1 | 2,6 | 5,7 |
| 205648_at | WNT2 | 2,6 | 30,2 |
| 230643_at | WNT9A | 2,3 | 3,2 |
| 210875_s_at | ZEB1 | 2,0 | 4,9 |
| 212758_s_at | ZEB1 | 1,8 | 6,3 |

The above data indicate that the transformation of an adenoma to a CRC coincides with the onset of expression of a TGF-beta-driven transcriptional program in CAFs. It was next investigated whether different degrees of stromal TGF-beta signaling in CRC were associated with clinical disease progression. A representative pooled cohort of 340 CRC cases treated at three different hospitals for which transcriptomic profiles of primary tumors and clinical follow-up were publicly available (see Methods) was interrogated. Gene Set Enrichment Analysis (GSEA) revealed a strong association of the F-TBRS with the two most relevant clinical progression parameters: metastatic dissemination at the time of diagnosis (combined AJCC stage III + IV vs. stage I + II; FDR < 10⁻⁶) and eventual cancer relapse (FDR < 10⁻⁶) (data not shown). These data imply that the transition from early to late stage CRC is characterized by high levels of the TGF-beta-induced genes in CAFs.

To further explore the link between TGF-beta signaling in CAFs and tumor recurrence, the CRC patient cohort was stratified into three groups according to low, medium or high average expression of F-TBRS genes (Fig. 6). Large differences in the relative risk of cancer relapse were observed between the three groups. During 10 years of follow-up, 55% CRC patients with F-TBRS highly positive primary tumors suffered tumor recurrence, whereas all patients with F-TBRS low tumors remained disease-free (Fig. 6).

Cancer recurs in approximately 20-30% of stage II and in 30-50% of stage III CRC patients undergoing intended curative therapy, commonly in the form of distant metastasis. F-TBRS levels were robustly associated with relapse in stage II and stage III patients and identified a small set of patients (10%) in both groups (F-TBRS low) with no observed recurrences (Fig. 7). Of note, even the rare relapses occurring in Stage I CRCs (2 out of 45 patients in this group) were associated with high levels of fibroblast-specific TGF-beta driven genes. Cox proportional hazards multivariate analysis (Table 2) demonstrated that F-TBRS expression is an independent predictor of cancer recurrence that outperformed AJCC staging system in the identification of CRC patients that remain disease-free upon therapy.

**Table 2: Multivariate analysis using Cox Proportional Hazards Model to assess dependency of F-TBRS and AJCC staging in the prediction of cancer relapse**

| | HR | 95% CI | P value |
|---|---|---|---|
| F-TBRS | | | 0.0001 |
| Medium vs. Low | >100 | (N/A - N/A)* | 0.0057 |
| High vs. Low | >100 | (N/A - N/A)* | <0.0001 |
| High vs. Medium | 2.02 | (1.09-3.74) | 0.0190 |
| AJCC Stage | | | 0.0002 |
| Stage 2 vs. 1 | 2.35 | (0.53 - 10.40) | 0.2103 |
| Stage 3 vs. 1 | 6.22 | (1.49 - 26.00) | 0.0009 |
| Stage 3 vs. 2 | 2.64 | (1.42 - 4.91) | 0.0012 |

| | | | |
|---|---|---|---|
| *N/A - Not Applicable. No patients developed recurrence in F-TBRS low group. This parameter cannot be calculated HR, Hazard Ratio Cl, Confidence Interval | | | |

### EXAMPLE 3

### Identification of mini-signatures capable of predicting outcome of CRC

The F-TBRS was further analyzed as described in Materials and Methods in order to identify the minimal subset of genes that provides good prediction of disease-free survival. A subset of three genes (minisignature) formed by the CDKN2B, NPR3/C5orf23, and FLT-1 genes was identified which associated with time to recurrence in a statistically significant manner in all patients analysed as well as in patients from stage II and stage III subgroups. Figure 8 shows Kaplan Meier curves wherein survival of patients is plotted depending on the average expression of the CDKN2B, NPR3/C5orf23 and FLT-1 genes in all patients (panel A), in stage II patients (panel B) or in stage III patients (panel C).

Moreover, as shown in figure 9, the slope of the three gene expression signature shows an approximate incremental linear relationship with the risk of recurrence.

When the patients were stratified according to the stage of the tumor, two additional signatures were identified that provided statistically significant prediction of time to recurrence. In stage II patients, the expression signature formed by CDKN2B, NPR3/C5orf23, FLT-1, FRMD6, IGFBP3 and ESM1 allowed prediction of time to recurrence with a p<0.0039 (Figure 10). In stage III patients, the expression signature formed by CDKN2B, NPR3/C5orf23, FLT-1, FGF1, GEM and MEX3B allowed prediction of time to recurrence with a p<0.0001 (figure 11). In both cases, the signature expression displayed an incremental and approximately linear effect on the risk of recurrence (figures 10B and 11B).

In addition, we studied the association of the 6 genes predictors in two independent cohorts of patients, GSE 33113 (Fig. 12) containing stage II CRC patients and GSE37892 (Fig. 13) containing both stage II and stage III CRC patients. Figure 12 shows Kaplan Meier curves wherein survival of patients is plotted depending on the average expression of the CDKN2B, NPR3/C5orf23, FLT-1, FRMD6, IGFBP3 and ESM1 genes in all patients (stage II) of GSE 33113 (Fig. 12A). For every increment (+1SD) in the average expression of the colostage II predictor there is a 1.47 increase in the risk to experience recurrence (Fig. 12B).

Figure 13 shows Kaplan Meier curves wherein survival of patients is plotted depending on the average expression of the CDKN2B, NPR3/C5orf23, FLT-1, FGF1, GEM and MEX3B genes in all patients of GSE 37892 (Fig. 13A). For every increment (+1SD) in the average expression of the colostage III predictor there is a 1.52 increase in the risk to experience recurrence (Fig. 13B).

### EXAMPLE 4

### TGF-beta 2 and TGF-beta 3 can predict recurrence in colorectal cancer (not part of the invention)

The expression levels of *TGFB2* and *TGFB3* had similar predictive power over disease relapse as the expression levels of the genes forming the F-TBRS (Fig. 14).

*TGFB2* and *TGFB3* are independent predictors for recurrence in colorectal cancer. As shown in figure 15, there is a correlation between the SCAD coefficient and the percentage of recurrence.

Since tumour stage is an information available to the oncologist at the time of diagnosis, the prognostic value of *TGFB2* and *TGFB3* levels in combination with staging was evaluated. Again, this allows for the identification of a group of patients at very low risk of disease recurrence (local or distant) in both groups.

| | **[<-1.5]** | **[-1-5,-1]** | **[-1,0]** | **[>0]** |
|---|---|---|---|---|
| **Stage I** | | | | |
| No recurrent | 43 | 0 | 0 | 0 |
| Recurrent | 1 | 1 | 0 | 0 |

| **Stage II** | | | | |
|---|---|---|---|---|
| No recurrent | 79 | 11 | 5 | 0 |
| Recurrent | 8 | 5 | 0 | 1 |

| **Stage III** | | | | |
|---|---|---|---|---|
| No recurrent | 19 | 17 | 28 | 9 |
| Recurrent | 2 | 5 | 23 | 7 |

| **Multivariate Cox Analysis** | | **HR** | **95% CI** | **P-value** |
|---|---|---|---|---|
| **TGF-beta 2** | | 1.57 | 1.05-2.35 | 0.0376 |
| | (+1SD) | 1.34 | 1.03-1.75 | 0.0376 |
| **AJCC STAGE** | | | | <0.0001 |
| **Stage 2 vs Stage 1** | | 3.02 | -1.46-13.35 | 0.0966 |
| **Stage 3 vs Stage 1** | | 8.16 | 1.96-33.95 | 0.0001 |
| **Stage 3 vs Stage 2** | | 2.70 | 1.45-5.03 | 0.0010 |

| **Multivariate Cox Analysis** | | **HR** | **95% CI** | **P-value** |
|---|---|---|---|---|
| **TGF-beta 3** | | 1.62 | 1.05-2.35 | 0.0376 |
| | (+1SD) | 1.43 | 1.03-1.75 | 0.0376 |
| **AJCC STAGE** | | | | <0.0001 |
| **Stage 2 vs Stage 1** | | 3.04 | -1.45-13.39 | 0.0939 |
| **Stage 3 vs Stage 1** | | 8.14 | 1.96-33.87 | 0.0001 |
| **Stage 3 vs Stage 2** | | 2.68 | 1.44-4.98 | 0.0010 |

Data supporting this finding is provided in Figure 15, wherein it is shown that patients with SCAD coefficients below -1.5 are at low risk to develop disease recurrence.

## Claims

1. A method for predicting the outcome of a patient suffering colorectal cancer, or for selecting a patient which is likely to benefit from adjuvant therapy after surgical resection of colorectal cancer comprising the determination of the expression levels of the NPR3/C5orf23, CDKN2B and FLT1 genes in a sample from said patient,
wherein an increased expression level of said genes with respect to a reference value for said genes is indicative of an increased likelihood of a negative outcome of the patient, or that the patient is likely to benefit from therapy after surgical treatment or
wherein a decreased expression level of said genes with respect to reference values for said genes is indicative of an increased likelihood of a positive outcome of the patient, or that the patient is unlikely to benefit from therapy after surgical treatment.

2. A method according to claim 1 additionally comprising the determination of the expression levels of one or more genes selected from the group consisting of group of FRMD6, IGFBP3, ESM1, FGF1, GEM, MEX3B, WNT2, NGF, MSC, SETBP1, FLJ10357, DACT, MURC and Col10A1
wherein an increased expression level of said genes with respect to a reference value for said genes is indicative of an increased likelihood of a negative outcome of the patient, or that the patient is likely to benefit from therapy after surgical treatment or
wherein a decreased expression level of said genes with respect to reference values for said genes is indicative of an increased likelihood of a positive outcome of the patient, or that the patient is unlikely to benefit from therapy after surgical treatment.

3. A method according to claim 2 wherein
(i) the expression levels of CDKN2B, NPR3/C5orf23, FLT1, FRMD6, IGFBP3 and ESM1 genes is determined and wherein the patient is a patient suffering from stage II colorectal cancer or.
(ii) the expression levels of the CDKN2B, NPR3/C5orf23, FLT1, FGF1, GEM, and MEX3B genes is determined and wherein the patient is a patient suffering from stage III colorectal cancer.

4. A method according to claim 2 comprising the determination of the expression levels of the ANGPTL2, ANGPTL4, APBB2, BMPR2, BPGM, C13orf33, C5orf13, NPR/C5orf23, CACHD1, CALD1, CDH6, CDKN2B, CILP, CNTN1, COL10A1, C0L12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A and ZEB1 genes and of the genes which hybridize specifically with the probes having the sequences SEQ ID NO:1 to 13,
wherein an increased expression level of said genes with respect to a reference value for said genes is indicative of an increased likelihood of a negative outcome of the patient, or that the patient is likely to benefit from therapy after surgical treatment or
wherein a decreased expression level of said genes with respect to reference values for said genes is indicative of an increased likelihood of a positive outcome of the patient, or that the patient is unlikely to benefit from therapy after surgical treatment.

5. A method according to any of claims 1 to 4 wherein the tumor stage in the patient is additionally determined and
wherein a high tumor stage is indicative of an increased likelihood of a negative outcome, or that the patient is likely to benefit from therapy after surgical treatment or
wherein a low tumor stage is indicative of an increased likelihood of a positive outcome, or that the patient is unlikely to benefit from therapy after surgical treatment.

6. A method according to any of claims 1 to 5 wherein the therapy is selected from the group consisting of chemotherapy, radiotherapy and/or a therapy comprising a TGF-beta inhibitor.

7. Method according to any of claims 1 to 6 wherein the outcome to be predicted is either recurrence or development of metastasis.

8. A method according to any of claims 1 to 7 wherein the sample is selected from the group consisting of a tumor biopsy or a bio fluid.

9. A chemotherapeutic agent for use in the treatment of colorectal cancer in a patient after surgical treatment of the cancer, wherein the patient has been selected by a method according to any of claims 1 to 8 and wherein the chemotherapeutic agent comprises a TGF-beta inhibitor..

10. A kit comprising reagents adequate for determining the expression levels of the NPR3/C5orf23, CDKN2B and FLT1 genes and, optionally, reagents for the determination of the expression levels of one or more housekeeping genes, wherein the reagents adequate for determining the expression levels of the NPR3/C5orf23, CDKN2B and FLT1 genes comprise at least 10% of the probes present in the kit.

11. A kit according to claim 10 further comprising reagents adequate for the determination of the expression levels of one or more genes selected from the group consisting to FRMD6, IGFBP3, ESM1, FGF1, GEM, MEX3B, WNT2, NGF, MSC, SETBP1, FLJ10357, DACT1, MURC and Col10A1.

12. A kit according to claim 11 wherein the reagents are adequate for the determination of the expression levels of the CDKN2B, NPR3/C5orf23, FLT1, FRMD6, IGFBP3 and ESM1 genes or of the CDKN2B, NPR3/C5orf23, FLT1, FGF1, GEM, and MEX3B genes.

13. A kit according to claim 12 wherein the reagents are adequate for the determination of the expression levels of the ANGPTL2, ANGPTL4, APBB2, BMPR2, BPGM, C13orf33, C5orf13, NPR/C5orf23, CACHD1, CALD1, CDH6, CDKN2B, CILP, CNTN1, COL10A1, C0L12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A and ZEB1 genes and of the genes which hybridize specifically with the probes having the sequences SEQ ID NO:1 to 13.

14. Use of a kit according to any of claims 10 to 13 for predicting the outcome of a patient suffering colorectal cancer, or for selecting a patient which is likely to benefit from adjuvant therapy after surgical resection of colorectal cancer.

## Patentansprüche

1. Verfahren zum Vorhersagen des Ergebnisses eines Patienten, der an Darmkrebs leidet, oder zum Auswählen eines Patienten, der wahrscheinlich von einer adjuvanten Therapie nach der chirurgischen Resektion von Darmkrebs profitieren wird, umfassend das Bestimmen des Expressionsniveaus der Gene NPR3/C5orf23, CDKN2B und FLT1 in einer Probe des besagten Patienten,
wobei ein erhöhtes Expressionsniveau besagter Gene in Bezug auf einen Referenzwert für besagte Gene indikativ für eine erhöhte Wahrscheinlichkeit eines negativen Ergebnisses des Patienten ist, oder dass der Patient wahrscheinlich von einer Therapie nach einer chirurgischen Behandlung profitieren wird, oder
wobei ein vermindertes Expressionsniveau besagter Gene in Bezug auf Referenzwerte für besagte Gene indikativ für eine erhöhte Wahrscheinlichkeit eines positiven Ergebnisses des Patienten ist, oder dass der Patient nach einer chirurgischen Behandlung wahrscheinlich nicht von einer Therapie profitieren wird.

2. Verfahren nach Anspruch 1, das zusätzlich das Bestimmen der Expressionsniveaus eines oder mehrerer Gene umfasst, die aus der Gruppe ausgewählt sind, bestehend aus der Gruppe aus FRMD6, IGFBP3, ESM1, FGF1, GEM, MEX3B, WNT2, NGF, MSC, SETBP1, FLJ10357, DACT, MURC und Col10A1
wobei ein erhöhtes Expressionsniveau besagter Gene in Bezug auf einen Referenzwert für besagte Gene indikativ für eine erhöhte Wahrscheinlichkeit eines negativen Ergebnisses des Patienten ist, oder dass der Patient wahrscheinlich von einer Therapie nach einer chirurgischen Behandlung profitieren wird, oder
wobei ein vermindertes Expressionsniveau besagter Gene in Bezug auf Referenzwerte für besagte Gene indikativ für eine erhöhte Wahrscheinlichkeit eines positiven Ergebnisses des Patienten ist, oder dass der Patient nach der chirurgischen Behandlung wahrscheinlich nicht von einer Therapie profitieren wird.

3. Verfahren nach Anspruch 2, wobei
(i) die Expressionsniveaus der Gene CDKN2B, NPR3/C5orf23, FLT1, FRMD6, IGFBP3 und ESM1 bestimmt werden und wobei der Patient ein Patient ist, der an Darmkrebs im Stadium II leidet, oder
(ii) die Expressionsniveaus der Gene CDKN2B, NPR3/C5orf23, FLT1, FGF1, GEM und MEX3B bestimmt werden und wobei der Patient ein Patient ist, der an Darmkrebs im Stadium III leidet.

4. Verfahren nach Anspruch 2, umfassend das Bestimmen der Expressionsniveaus der Gene ANGPTL2, ANGPTL4, APBB2, BMPR2, BPGM, C13orf33, C5orfl3, NPR/C5orf23, CACHD1, CALD1, CDH6, CDKN2B, CILP, CNTN1, COL10A1, C0L12A1, COL27A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KALI, KIAA1755, KLF7, LARP6, LMCD1, LM04, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A und ZEB1 und der Gene, die spezifisch mit den Sonden mit den Sequenzen SEQ ID NO:1 bis 13 hybridisieren,
wobei ein erhöhtes Expressionsniveau besagter Gene in Bezug auf einen Referenzwert für besagte Gene indikativ für eine erhöhte Wahrscheinlichkeit eines negativen Ergebnisses des Patienten ist, oder dass der Patient wahrscheinlich von einer Therapie nach einer chirurgischen Behandlung profitiert, oder
wobei ein verminderter Expressionsgrad der Gene in Bezug auf die Referenzwerte für besagte Gene indikativ für eine erhöhte Wahrscheinlichkeit eines positiven Ergebnisses des Patienten ist, oder dass der Patient nach einer chirurgischen Behandlung wahrscheinlich nicht von einer Therapie profitieren wird.

5. Verfahren nach einem der Ansprüche 1 bis 4, wobei das Tumorstadium im Patienten zusätzlich bestimmt wird und
wobei ein hohes Tumorstadium indikativ für eine erhöhte Wahrscheinlichkeit eines negativen Ergebnisses ist, oder dass der Patient wahrscheinlich von einer Therapie nach einer chirurgischen Behandlung profitieren wird, oder
wobei eine niedrige Tumorstufe indikativ eine erhöhte Wahrscheinlichkeit eines positiven Ergebnisses ist, oder dass der Patient nach der chirurgischen Behandlung wahrscheinlich nicht von einer Therapie profitieren wird.

6. Verfahren nach einem der Ansprüche 1 bis 5, wobei die Therapie ausgewählt ist aus der Gruppe bestehend aus Chemotherapie, Strahlentherapie und/oder einer Therapie, die einen TGF-beta-Inhibitor umfasst.

7. Verfahren nach einem der Ansprüche 1 bis 6, wobei das vorherzusagende Ergebnis entweder ein Wiederauftreten oder die Entwicklung von Metastasen ist.

8. Verfahren nach einem der Ansprüche 1 bis 7, wobei die Probe ausgewählt ist aus der Gruppe bestehend aus einer Tumorbiopsie oder einer Bioflüssigkeit.

9. Chemotherapeutikum zur Verwendung bei der Behandlung von Darmkrebs bei einem Patienten nach der chirurgischen Behandlung des Krebses, wobei der Patient durch ein Verfahren nach einem der Ansprüche 1 bis 8 ausgewählt wurde und wobei das Chemotherapeutikum einen TGF-beta-Inhibitor umfasst.

10. Kit, umfassend Reagenzien, die zum Bestimmen der Expressionsniveaus der Gene NPR3/C5orf23, CDKN2B und FLT1 geeignet sind, und gegebenenfalls Reagenzien zum Bestimmen der Expressionsniveaus eines oder mehrerer Haushaltsgene, wobei die Reagenzien, die zum Bestimmen der Expressionsniveaus der Gene NPR3/C5orf23, CDKN2B und FLT1 geeignet sind, mindestens 10% der im Kit vorhandenen Sonden umfassen.

11. Kit nach Anspruch 10, ferner umfassend Reagenzien, die für die Bestimmung der Expressionsniveaus eines oder mehrerer Gene aus der Gruppe bestehend aus FRMD6, IGFBP3, ESM1, FGF1, GEM, MEX3B, WNT2, NGF, MSC, SETBP1, FLJ10357, DACT1, MURC und Col10A1 geeignet sind.

12. Kit nach Anspruch 11, wobei die Reagenzien zur Bestimmung der Expressionsniveaus der Gene CDKN2B, NPR3/C5orf23, FLT1, FRMD6, IGFBP3 und ESM1 oder der Gene CDKN2B, NPR3/C5orf23, FLT1, FGF1, GEM und MEX3B ausreichend sind.

13. Kit nach Anspruch 12, wobei die Reagenzien für die Bestimmung der Expressionsniveaus von ANGPTL2, ANGPTL4, APBB2, BMPR2, BPGM, C13orf33, C5orf13, NPR/C5orf23, CACHD1, CALD1, CDH6, CDKN2B, CILP, CNTN1, COL10A1, C0L12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KALI, KIAA1755, KLF7, LARP6, LMCD1, LM04, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A und ZEB1 und der Gene geeignet sind, die spezifisch mit den Sonden mit den Sequenzen SEQ ID NO:1 bis 13 hybridisieren.

14. Verwendung eines Kits nach einem der Ansprüche 10 bis 13 zum Vorhersagen des Ergebnisses eines an Darmkrebs erkrankten Patienten oder zum Auswählen eines Patienten, der nach der chirurgischen Resektion von Darmkrebs wahrscheinlich von einer adjuvanten Therapie profitieren wird.

## Revendications

1. Un procédé pour prédire le résultat d'un patient souffrant d'un cancer colorectal, ou pour sélectionner un patient qui est susceptible de bénéficier d'une thérapie adjuvante après résection chirurgicale du cancer colorectal, comprenant la détermination des niveaux d'expression des gènes NPR3/C5orf23, CDKN2B et FLT1 dans un échantillon provenant dudit patient,
un niveau d'expression accru desdits gènes par rapport à une valeur de référence pour lesdits gènes étant indicatif d'une probabilité accrue d'un résultat négatif pour le patient, ou du fait que le patient est susceptible de bénéficier d'une thérapie après une traitement chirurgical ou
un niveau d'expression diminué desdits gènes par rapport aux valeurs de référence desdits gènes étant indicatif d'une probabilité accrue d'un résultat positif pour le patient, ou du fait que le patient est peu susceptible de bénéficier d'une thérapie après traitement chirurgical.

2. Un procédé selon la revendication 1 comprenant en outre la détermination des niveaux d'expression d'un ou plusieurs gènes choisis dans le groupe consistant en le groupe de FRMD6, IGFBP3, ESM1, FGF1, GEM, MEX3B, WNT2, NGF, MSC, SETBP1, FLJ10357, DACT, MURC et Col10A1
un niveau d'expression accru desdits gènes par rapport à une valeur de référence desdits gènes étant indicatif d'une probabilité accrue de résultat négatif chez le patient, ou du fait que le patient est susceptible de bénéficier d'une thérapie après une traitement chirurgical ou
un niveau d'expression diminué desdits gènes par rapport aux valeurs de référence desdits gènes étant indicatif d'une probabilité accrue d'un résultat positif pour le patient, ou du fait que le patient est peu susceptible de bénéficier d'une thérapie après traitement chirurgical.

3. Un procédé selon la revendication 2 dans lequel
(i) les niveaux d'expression des gènes CDKN2B, NPR3/C5orf23, FLTI, FRMD6, IGFBP3 et ESM1 sont déterminés et le patient est un patient souffrant de cancer colorectal de stade II ou
(ii) les niveaux d'expression des gènes CDKN2B, NPR3/C5orf23, FLT1, FGF1, GEM et MEX3B sont déterminés et le patient est un patient souffrant de cancer colorectal de stade III.

4. Un procédé selon la revendication 2 comprenant la détermination des niveaux d'expression des ANGPTL2, ANGPTL4, APBB2, BMPR2, BPGM, C13orf33, C5orf13, NPR/C5orf23, CACHD1, CALD1, CDH6, CDKN2B, CILP, CNTN1, COL10A1, C0L12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPM1E, PTHLH, RASD1, RASGRP3, RASL12, RGS4, RNF150, RUNX1, S1PR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A et ZEB1 et des gènes qui s'hybride spécifiquement avec les sondes ayant les séquences SEQ ID NO :1 à 13,
un niveau d'expression accru desdits gènes par rapport à une valeur de référence pour lesdits gènes étant indicatif d'une probabilité accrue d'un résultat négatif pour le patient, ou du fait que le patient est susceptible de bénéficier d'une thérapie après une traitement chirurgical ou
un niveau d'expression diminué desdits gènes par rapport aux valeurs de référence pour lesdits gènes étant indicatif d'une probabilité accrue de résultat positif pour le patient ou du fait que le patient est peu susceptible de bénéficier du traitement après traitement chirurgical.

5. Un procédé selon l'une quelconque des revendications 1 à 4, dans lequel le stade de la tumeur chez le patient est en outre déterminé et
dans lequel un stade tumoral élevé indique une probabilité accrue d'un résultat négatif, ou que le patient est susceptible de bénéficier d'une thérapie après un traitement chirurgical ou
dans lequel un stade tumoral faible indique une probabilité accrue d'un résultat positif, ou que le patient est peu susceptible de bénéficier d'une thérapie après un traitement chirurgical.

6. Un procédé selon l'une quelconque des revendications 1 à 5, dans lequel la thérapie est choisie dans le groupe constitué par la chimiothérapie, la radiothérapie et/ou une thérapie comprenant un inhibiteur du TGF-bêta.

7. Procédé selon l'une quelconque des revendications 1 à 6, dans lequel le résultat à prédire est soit la récurrence, soit le développement de métastases.

8. Un procédé selon l'une quelconque des revendications 1 à 7, dans lequel l'échantillon est choisi dans le groupe constitué par une biopsie tumorale ou un biofluide.

9. Un agent chimiothérapeutique destiné à être utilisé dans le traitement du cancer colorectal chez un patient après un traitement chirurgical du cancer, le patient ayant été choisi par un procédé selon l'une quelconque des revendications 1 à 8 et l'agent chimiothérapeutique comprenant un inhibiteur du TGF-beta.

10. Un kit comprenant des réactifs adéquats pour déterminer les niveaux d'expression des gènes NPR3/C5orf23, CDKN2B et FLT1 et, facultativement, des réactifs pour la détermination des niveaux d'expression d'un ou plusieurs gènes domestiques, les réactifs adéquats pour déterminer les niveaux d'expression des gènes NPR3/C5orf23, CDKN2B et FLT1 comprennent au moins 10% des sondes présentes dans le kit.

11. Un kit selon la revendication 10 comprenant en outre des réactifs adéquats pour la détermination des niveaux d'expression d'un ou plusieurs gènes choisis dans le groupe constitué par FRMD6, IGFBP3, ESM1, FGF1, GEM, MEX3B, WNT2, NGF, MSC, SETBP1, FLJ10357, DACT1, MURC et Col10A1.

12. Un kit selon la revendication 11, dans lequel les réactifs sont adéquats pour la détermination des niveaux d'expression des gènes CDKN2B, NPR3/C5orf23, FLT1, FRMD6, IGFBP3 et ESM1 ou des gènes CDKN2B, NPR3/C5orf23, FLT1, FGF1, GEM et MEX3B.

13. Un kit selon la revendication 12, dans lequel les réactifs sont adéquats pour la détermination des niveaux d'expression des ANGPTL2, ANGPTL4, APBB2, BMPR2, BPGM, C13orf33, C5orfl3, NPR/C5orf23, CACHD1, CALD1, CDH6, CDKN2B, CILP, CNTN1, COL10A1, C0L12A1, COL27A1, DACT1, DIXDC1, DNAJB5, DNAJC18, ELTD1, EPHA4, ESM1, FAP, FGD6, FGF1, FGF2, FLJ10357, FLT-1, FN1, FRMD4A, FRMD6, GAS1, GEM, GFPT2, GPR161, HAS2, HEY1, HIC1, HS3ST3A1, IGFBP3, IGFBP7, IL11, INHBA, KAL1, KIAA1755, KLF7, LARP6, LMCD1, LMO4, LOC100128178, LOC644242, LOC728264, LOH3CR2A, LRRC8A, MEOX1, MEX3B, MFAP2, MGC16121, MSC, MURC, NEDD9, NGF, NOX4, NPR2, NUAK1, OSGIN2, PALLD, PALM2, PDGFA, PDGFC, PDLIM4, PDPN, PGM2L1, PKNOX2, PMEPA1, PODXL, PPMIE, PTHLH, RASD1, RASGRP3, RASL12, RGS4, RNF150, RUNX1, SIPR5, SEMA6D, SERPINE1, SETBP1, SHISA2, SLC46A3, SNCAIP, SNORD114-3, SOX6, SPSB1, STK38L, SYNE1, SYTL4, TCF4, TGFB2, TIMP3, TMEM88, TNC, TNS1, TPM1, TSHZ3, TSPAN2, VEPH1, WNT2, WNT9A et ZEB1 et des gènes qui s'hybride spécifiquement avec les sondes ayant les séquences SEQ ID NO : 1 à 13.

14. Utilisation d'un kit selon l'une quelconque des revendications 10 à 13 pour prédire le résultat d'un patient souffrant d'un cancer colorectal, ou pour sélectionner un patient qui est susceptible de bénéficier d'une thérapie adjuvante après résection chirurgicale du cancer colorectal.
